# EUROPÄISCHE PATENTSCHRIFT

(11) **EP 3 713 520 B1**
(45) Veröffentlichungstag und Bekanntmachung des Hinweises auf die Patenterteilung: **26.01.2022**
(21) Anmeldenummer: 18795980.4
(22) Anmeldetag: 26.10.2018
(51) Int. Cl.: A61F 2/28, A61F 2/30, A61F 2/34, A61F 2/36, A61F 2/38

(54) **IMPLANTAT ZUM BEHANDELN UND/ODER BIOLOGISCHEN REKONSTRUIEREN EINES KNOCHENDEFEKTS**
IMPLANT FOR THE TREATMENT AND/OR BIOLOGICAL RECONSTRUCTION OF A BONE DEFECT
IMPLANT POUR LE TRAITEMENT ET/OU LA RECONSTRUCTION BIOLOGIQUE D'UN DÉFAUT OSSEUX

(30) Priorität: 20.11.2017 DE 102017220710
(43) Veröffentlichungstag der Anmeldung: 30.09.2020
(73) Patentinhaber: Aesculap AG, 78532 Tuttlingen (DE)
(72) Erfinder: HETTICH, Georg, 78532 Tuttlingen (DE); KÖNIG, Silke, Rottweil 78628 (DE); ABELE, Wolfgang, Tuttlingen 78532 (DE)
(74) Vertreter: Vigand, Philippe
(86) Internationale Anmeldenummer: PCT/EP2018/079383
(87) Internationale Veröffentlichungsnummer: WO 2019/096561

(56) Entgegenhaltungen:
- WO-A1-2012/061024
- WO-A2-2008/021921
- DE-A1-102012 213 246
- US-A1- 2007 142 916

## Beschreibung

Die Erfindung betrifft ein Implantat sowie ein Kit zum Behandeln und/oder biologischen Rekonstruieren eines Knochendefekts, eine Umhüllung für osteokonduktive Stützkörper, die Verwendung eines in Wasser oder einer wasserhaltigen Flüssigkeit löslichen Materials zur Herstellung einer Umhüllung für osteokonduktive Stützkörper und ein Verfahren zum Behandeln und/oder biologischen Rekonstruieren eines Knochendefekts.

Große Knochendefekte am Azetabulum stellen bei Revisionsoperationen am Hüftgelenk ein schwerwiegendes Problem dar. Bei einer Hüftrevisionsoperation werden hauptsächlich die drei nachfolgend genannten Ziele verfolgt.

Zunächst soll das ursprüngliche Gelenkzentrum wiederhergestellt werden.

Weiterhin wird eine stabile Fixierung eines zum Behandeln eines Azetabulumdefekts eingesetzten Implantats angestrebt, wobei zwischen einer sogenannten Primärstabilität und einer sogenannten Sekundärstabilität unterschieden wird.

Unter der Primärstabilität wird die auf Reibung oder Befestigungselementen, wie beispielsweise Knochenschrauben, beruhende Fixierung des Implantats in den ersten Wochen nach einer Operation verstanden. Unter der Sekundärstabilität wird die auf knöcherne Anwachsungen beruhende Fixierung des Implantats verstanden. Die Sekundärstabilität setzt in der Regel etwa einen Monat nach einem operativen Eingriff ein und kann bis zu mehrere Jahre andauern.

Schließlich wird eine sogenannte biologische Rekonstruktion des knöchernen Defekts angestrebt. Unter einer biologischen Rekonstruktion werden die Durchdringung und/oder der Wiederaufbau eines Knochendefekts mit körpereigenem Knochen verstanden.

Azetabulumdefekte werden als geschlossene Defekte ("contained"-Defekte) bezeichnet, wenn vitaler Patientenknochen und eine eingebrachte Hüftpfanne den Defekt vollständig umschließen. Dagegen werden Azetabulumdefekte als offene Defekte ("uncontained"-Defekte) bezeichnet, wenn eine eingebrachte Hüftpfanne den Defekt nicht vollständig umschließt, d.h. offene Stellen zurückbleiben.

Für die Behandlung geschlossener Azetabulumdefekte hat sich in der Praxis die sogenannte "Impaction Bone Grafting"-Technik durchgesetzt. Bei dieser Technik werden aus einem allogenen Knochenmaterial (Femurkopf) etwa 0,5 cm große Knochenstücke (Spongiosa) hergestellt und mittels eines geeigneten Instruments in den Knochendefekt verdichtet. Mit Hilfe dieser Technik kann der Azetabulumdefekt biologisch rekonstruiert und das Rotationszentrum wiederhergestellt werden. Allerdings bedeuten allogene Knochenchips trotz Thermobehandlung ein Infektionsrisiko. Einen weiteren Nachteil stellen die Kosten für die Herstellung von allogenen Knochenchips dar, welche aufgrund erhöhter regulatorischer Anforderungen deutlich gestiegen sind. Schließlich ist die intra-operative Handhabung äußerst kompliziert und zeitintensiv. So muss der humane Femurkopf, aus welchem die Knochenchips hergestellt werden, während der Operation aufgetaut und zerkleinert werden. Die auf diese Weise hergestellten Chips sind unregelmäßig geformt, und es bedarf einiger chirurgischer Erfahrung, damit die Chips richtig benutzt werden.

Einen weiteren Ansatz zur Behandlung von Knochendefekten stellen künstliche Knochenersatzmaterialien, insbesondere auf Basis von Calciumphosphatmaterialien, dar. Nachteilig bei künstlichen Knochenersatzmaterialien ist häufig ihre begrenzte mechanische Belastbarkeit, d.h. die mit ihnen erzielbare Primärstabilität ist begrenzt. Ein weiterer Nachteil besteht in der Gefahr eines Abbaus oder einer Resorption der Knochenersatzmaterialien ohne Knochenwachstum und mithin ohne Erzielung einer Sekundärstabilität.

Aus der US 8,562,613 B2 ist ein Kit zur Behandlung von Knochendefekten mit einer Mischung aus einem osteokonduktiven Material und einem osteoinduktiven Material sowie einem porösen Container bekannt.

Gegenstand der EP 0 764 008 B1 ist eine Vorrichtung zur Verwendung bei der Stabilisierung eines spinalen Bewegungssegments mit einem flexiblen Beutel, wobei der Beutel ein biologisches Füllmaterial zur Förderung einer Knochen- oder Faserverwachsung enthalten kann.

Die EP 1 408 888 B1 offenbart ein System zur Korrektur von Wirbelkompressionsfrakturen, welches einen porösen Beutel sowie ein Füllwerkzeug aufweist, wobei das Füllwerkzeug dazu ausgebildet ist, um ein Knochenfüllmaterial unter Druck in den porösen Beutel zu injizieren.

Aus der WO 2012/061024 A1 ist ein implantierbarer Container bekannt, welcher wenigstens teilweise demineralisierte und osteoinduktive Knochenpartikel enthält.

Aus der WO 2008/021921 A2 ist ein medizinisches Gerät in Form einer dünnen Platte mit Körnern aus einem kalziumhaltigen, osteokonduktiven Material, die durch eine dünne Bahn aus Polymermaterial miteinander verbunden sind, bekannt. Die Vorrichtung umfasst eine erste Fläche und eine zweite Fläche, wobei mindestens die erste Fläche mit Vorsprüngen bestückt ist, die freiliegende Bereiche des kalziumhaltigen, osteokonduktiven Materials darstellen, die von darunter liegenden Körnern gebildet werden. Methoden zur Herstellung und Verwendung solcher Vorrichtungen werden ebenso beschrieben wie Verfahren und Vorrichtungen, bei denen sehr dünne Polymerfolien verwendet werden, die an die Knochenwachstumsflächen von lasttragenden orthopädischen Implantaten angepasst sind.

Die Publikation "Bone Regeneration by the Combined Use of Tetrapod-Shaped Calcium Phosphate Granules with Basic Fibroblast Growth Factor-Binding Ion Complex Gel in Canine Segmental Radial Defects (J. Vet. Med. Sci. 76(7):955-961, 2014)" von Honnami et al. beschäftigt sich mit einer Kombination von tetrapodenförmigem Granulat aus alpha-Tricalciumphosphat und einem osteoinduktiven Gel.

### AUFGABE UND LÖSUNG

Aufgabe der Erfindung ist es, ein Implantat bereitzustellen, welches sich zum Behandeln und/oder biologischen Rekonstruieren eines Knochendefekts, insbesondere periprothetischen Knochendefekts wie eines Azetabulumdefekts, eignet und die einleitend genannten Nachteile, insbesondere im Zusammenhang einer Hüftrevisionsoperation, vermeidet oder wenigstens teilweise vermeidet. Das Implantat soll insbesondere die Erzielung einer Primärstabilität und/oder einer Sekundärstabilität und/oder einer biologischen Rekonstruktion optimieren und eine einfache und komplikationsarme Handhabung ermöglichen.

Weiterhin ist es Aufgabe der Erfindung, ein Kit zum Behandeln und/oder biologischen Rekonstruieren eines Knochendefekts, eine Umhüllung für osteokonduktive Stützkörper, die Verwendung eines Materials zur Herstellung einer solchen Umhüllung sowie ein Verfahren zum Behandeln und/oder biologischen Rekonstruieren eines Knochendefekts bereitzustellen.

Diese Aufgaben werden durch ein Implantat mit den Merkmalen gemäß unabhängigem Anspruch 1 und durch ein in der Beschreibung offenbartes Kit, eine in der Beschreibung offenbarte Umhüllung, eine in der Beschreibung offenbarte Verwendung sowie durch ein in der Beschreibung offenbartes Verfahren zum Behandeln und/oder biologischen Rekonstruieren eines Knochendefekts gelöst. Bevorzugte Ausgestaltungen sind in den abhängigen Ansprüchen sowie in der Beschreibung definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung ein Implantat, vorzugsweise zum Behandeln und/oder biologischen Rekonstruieren, insbesondere Auskleiden und/oder Abdichten und/oder Unterfüttern und/oder wenigstens teilweisen Auffüllen, eines Knochendefekts.

Das Implantat kann im Sinne der vorliegenden Erfindung auch als Knochenersatzmaterial bezeichnet werden.

Das Implantat weist Folgendes auf:
- osteokonduktive Stützkörper und
- eine Umhüllung, welche die osteokonduktiven Stützkörper wenigstens teilweise, vorzugsweise vollständig, umgibt.

Die Umhüllung zeichnet sich besonders dadurch aus, dass sie ein Umhüllungsmaterial aufweist oder aus einem Umhüllungsmaterial besteht, welches in Wasser oder in einer wasserhaltigen Flüssigkeit löslich ist, wobei die Umhüllung Teilbereiche aufweist, welche unterschiedlich gestaltet sind. Ein solches Umhüllungsmaterial wird nachfolgend auch als lösliches Umhüllungsmaterial abgekürzt.

Die Umhüllung kann eine Folie, ein Vlies, ein Vliesstoff, ein Netz oder ein textiles Flächengebilde, insbesondere ein Gewebe, Geflecht oder eine Maschenware, wie beispielsweise ein gewirktes oder gestricktes Netz, sein. Die Umhüllung kann insbesondere durch Extrusion, durch Blasformen, durch Spritzguss (injection molding), durch einen Umformprozess (compression molding) oder durch ein additives Verfahren hergestellt oder gefertigt sein. Die Umhüllung kann weiterhin monofiles Fadenmaterial und/oder multifiles Fadenmaterial aufweisen oder aus monofilem Fadenmaterial und/oder multifilem Fadenmaterial bestehen.

Der Ausdruck "Knochendefekt" bedeutet im Sinne der vorliegenden Erfindung einen durch Verlust von Knochengewebe, insbesondere Gelenksknochengewebe, vorzugsweise Hüftgelenks- oder Kniegelenksknochengewebe, oder von Wirbelkörpergewebe betroffenen Knochenbereich, insbesondere Gelenksknochenbereich, vorzugsweise Hüftgelenks- oder Kniegelenksknochenbereich, oder Wirbelkörperbereich. Der Knochenverlust kann die Folge einer Knochenfraktur, eines Knochentraumas, einer Knochenerkrankung wie Tumorerkrankung oder einer chirurgischen Intervention/Reintervention, insbesondere einer Revision nach einer totalen Hüft- oder Kniearthroplastik, sein. Bevorzugt bedeutet der Ausdruck "Knochendefekt" im Sinne der vorliegenden Erfindung einen periprothetischen Knochendefekt, d.h. einen durch periprothetischen Knochengewebsverlust, insbesondere aufgrund von mechanischer Überlastung und/oder abrieb-induzierter Osteolyse und/oder Implantatmigration, betroffenen Knochenbereich.

Vorzugsweise handelt es sich bei dem Knochendefekt um einen Gelenksknochendefekt, insbesondere Kniegelenksknochendefekt oder Hüftgelenksknochendefekt, vorzugsweise Azetabulumdefekt, insbesondere geschlossenen oder offenen Azetabulumdefekt.

Weiterhin kann der Ausdruck "Knochendefekt" im Sinne der vorliegenden Erfindung einen menschlichen Knochendefekt oder einen tierischen Knochendefekt bedeuten.

Unter dem Ausdruck "tierischer Knochendefekt" soll im Sinne der vorliegenden Erfindung der Knochendefekt eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "Stützkörper" bedeutet im Sinne der vorliegenden Erfindung Körper, insbesondere regelmäßig und/oder unregelmäßig geformte Körper, welche dazu ausgebildet sind, in einem zu behandelnden und/oder biologisch zu rekonstruierenden Knochendefekt üblicherweise auftretenden Kräften ohne Deformation oder Zerstörung, wenigstens jedoch ohne wesentliche Deformation oder Zerstörung, zu widerstehen und mithin lasttragende Funktionen zu übernehmen. Daher können die Stützkörper im Sinne der vorliegenden Erfindung auch als osteokonduktive, lasttragende Stützkörper bezeichnet werden.

Der im Zusammenhang mit den Stützkörpern verwendete Ausdruck "osteokonduktiv" bedeutet im Sinne der vorliegenden Erfindung die Fähigkeit der Stützkörper zur Ausbildung einer dreidimensionalen Struktur, insbesondere Leitstruktur, oder einer dreidimensionalen Matrix, insbesondere Leitmatrix, welche ein Einwachsen von Knochengewebe, insbesondere Knochenneugewebe, begünstigt.

Der Ausdruck "in vivo abbaubar" bezieht sich im Sinne der vorliegenden Erfindung auf einen Stoff oder ein Material, welcher bzw. welches in einem menschlichen oder tierischen Körper, insbesondere unter der Einwirkung von Enzymen, metabolisiert werden kann. Der Abbau des Stoffs bzw. Materials kann dabei vollständig bis zur Mineralisierung, d.h. Freisetzung von chemischen Elementen und deren Einbau in anorganische Verbindungen, wie beispielsweise Kohlendioxid und/oder Sauerstoff und/oder Ammoniak, verlaufen oder auf der Stufe von abbaustabilen Zwischen- oder Transformationsprodukten stehen bleiben.

Unter dem Ausdruck "tierischer Körper" soll im Sinne der vorliegenden Erfindung der Körper eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "in vivo resorbierbar" bezieht sich im Sinne der vorliegenden Erfindung auf einen Stoff oder ein Material, welcher bzw. welches in einem menschlichen oder tierischen Körper durch lebende Zellen oder lebendes Gewebe, wie beispielsweise Nieren, aufgenommen werden kann, ohne dass ein Abbau oder ein nennenswerter Abbau des Stoffs bzw. Materials stattfindet.

Unter dem Ausdruck "Umhüllung" soll im Sinne der vorliegenden Erfindung ein Gebilde oder Konstrukt verstanden werden, welches dazu ausgebildet ist, (wenigstens) die osteokonduktiven Stützkörper wenigstens teilweise, vorzugsweise vollständig, zu umgeben oder zu umschließen. Hierzu weist die Umhüllung bevorzugt einen Hohlraum auf, welcher (wenigstens) mit den osteokonduktiven Stützkörpern wenigstens teilweise, bevorzugt nur teilweise, befüllbar oder befüllt ist.

Unter dem Ausdruck "Umhüllungsmaterial" soll im Sinne der vorliegenden Erfindung ein Material verstanden werden, welches Bestandteil der Umhüllung und insbesondere am Aufbau der Umhüllung beteiligt ist. Abhängig von seiner Löslichkeit in Wasser oder einer wasserhaltigen Flüssigkeit kann das Umhüllungsmaterial dabei Hauptbestandteil oder gar ausschließlicher Bestandteil der Umhüllung oder aber nur einen Nebenbestandteil, insbesondere im Sinne eines Additivs (Zusatzstoff), darstellen.

Unter dem Ausdruck "wasserhaltige Flüssigkeit" soll im Sinne der vorliegenden Erfindung eine wässrige Flüssigkeit verstanden werden, d.h. eine Flüssigkeit, welche Wasser enthält, wobei die Flüssigkeit optional weitere Stoffe, wie beispielsweise Salze, Proteine, Polysaccharide, Lipide, Blutbestandteile oder Mischungen davon, und/oder Zellen enthalten kann. Dementsprechend kann es sich bei der wasserhaltigen Flüssigkeit im Sinne der vorliegenden Erfindung beispielsweise um eine wässrige Dispersion, eine wässrige Lösung wie wässrige Salzlösung, eine wässrige Suspension oder um eine Körperflüssigkeit handeln. Bevorzugt soll unter dem Ausdruck "wasserhaltige Flüssigkeit" im Sinne der vorliegenden Erfindung eine wässrige Lösung oder eine Körperflüssigkeit, wie beispielsweise Blut und/oder Gewebeflüssigkeit und/oder Lymphflüssigkeit, verstanden werden. Weiterhin ist es bevorzugt, wenn die wasserhaltige Flüssigkeit im Sinne der vorliegenden Erfindung frei von organischen Lösungsmitteln ist.

Die vorliegende Erfindung zeichnet sich insbesondere durch die folgenden Vorteile aus:
- Die Umhüllung dient mit besonderem Vorteil als Einbringhilfe für die osteokonduktiven Stützkörper, ohne eine Barriere für einwachsendes Knochengewebe und mithin für eine biologische Rekonstruktion des knöchernen Defekts darzustellen. Durch das lösliche Umhüllungsmaterial wird mit besonderem Vorteil die Voraussetzung geschaffen, dass sich die Umhüllung nach Platzierung des Implantats in einen Knochendefekt wenigstens teilweise auflöst, so dass bei gutem Heilungsverlauf einwachsendes Knochengewebe die osteokonduktiven Stützkörper durchdringen kann, wodurch Letztere in den Knochen integriert werden können. Dadurch ist eine biologische Rekonstruktion und mithin eine Verkleinerung des knöchernen Defekts auch im Falle von nicht oder nur sehr langsam in vivo abbaubaren oder in vivo resorbierbaren osteokonduktiven Stützkörpern möglich.
   Bei fehlendem oder schlechtem Knochenwachstum bleiben die osteokonduktiven Stützkörper vorzugsweise erhalten und tragen damit zu einer Sekundärstabilität bei. Somit kann bevorzugt eine Sekundärstabilität gewährleistet werden, und zwar unabhängig davon, ob die osteokonduktiven Stützkörper nach Implantation mit Knochengewebe durchbaut werden oder nicht. Dies ist insbesondere im Hinblick auf die Versorgung älterer Patienten von Vorteil, bei welchen ein Knochenwachstum häufig überhaupt nicht mehr oder aber nur geringfügig stattfindet.
- Ein weiterer Vorteil der Umhüllung besteht darin, dass sie eine Ortsbindung der osteokonduktiven Stützkörper bewirkt. Dadurch lässt sich in besonders vorteilhafter Weise ein unkontrolliertes Einbringen der Stützkörper in den Knochendefekt vermeiden.
- Ein weiterer Vorteil der Umhüllung besteht insbesondere darin, dass ein Verdichten, insbesondere Impaktieren, der osteokonduktiven Stützkörper möglich ist, ohne dass es während des Verdichtens, insbesondere Impaktierens, zu einer unkontrollierten Verteilung der osteokonduktiven Stützkörper innerhalb des Knochendefekts kommt.
- Die Umhüllung bietet ferner den Vorteil einer einfachen und insbesondere schnellen Handhabung während eines operativen Eingriffs.
- Ein weiterer Vorteil besteht darin, dass die osteokonduktiven Stützkörper, insbesondere in Form eines losen oder vereinzelten Schüttguts, in einen verdichteten, bevorzugt impaktierten (eingeklemmten oder verkeilten), Zustand überführbar sind und in diesem Zustand als Leitstruktur für das Einwachsen von Knochengewebe, insbesondere neuem Knochengewebe, wirken können.
- Ein weiterer Vorteil besteht darin, dass das Implantat, insbesondere wenn die osteokonduktiven Stützkörper in einem verdichteten, insbesondere impaktierten, Zustand vorliegen, dauerhaft und vor allem homogen belastet werden kann. Eine homogene Implantatbelastung stellt eine Grundvoraussetzung für Knochenwachstum dar. Beispielsweise kann das Implantat mit verdichtet, insbesondere impaktiert, vorliegenden osteokonduktiven Stützkörpern dauerhaft mit einer Druckbelastung von bis zu 5 MPa beaufschlagt werden. Dabei kann eine durch Verdichten, insbesondere Impaktieren, erzeugte und von den osteokonduktiven Stützkörpern aufgebaute Struktur mit besonderem Vorteil eine elastische Verformung, insbesondere von 5 % bis 15 %, und ein geringes E-Modul, insbesondere von 50 MPa bis 300 MPa, aufweisen.
   Vorzugsweise weisen die osteokonduktiven Stützkörper wenigstens eine Abmessung oder Dimension in einem Größenbereich von 0,5 mm bis 5 mm, insbesondere 0,1 mm bis 3 mm, vorzugsweise 1 mm bis 2 mm, auf. Bei der wenigstens einen Abmessung bzw. Dimension kann es sich insbesondere um die Höhe und/oder Breite (Dicke) und/oder Länge und/oder den Durchmesser, insbesondere mittleren Durchmesser, der osteokonduktiven Stützkörper handeln.
   In einer weiteren Ausführungsform sind die osteokonduktiven Stützkörper gegeneinander beweglich, insbesondere gegeneinander verschiebbar.
   In einer weiteren Ausführungsform sind die osteokonduktiven Stützkörper impaktierbar, d.h. gegenseitig einklemmbar oder gegenseitig verkeilbar, ausgebildet.
   In einer weiteren Ausführungsform liegen die osteokonduktiven Stützkörper, d.h. gegenseitig eingeklemmt oder gegenseitig verkeilt, vor.
   In einer weiteren Ausführungsform sind die osteokonduktiven Stützkörper, bevorzugt mittels Impaktieren, in eine dreidimensionale und insbesondere Hohl- und/oder Zwischenräume aufweisende Struktur oder Matrix überführbar oder liegen in einer solchen Struktur bzw. Matrix vor. Eine solche Struktur bzw. Matrix kann im Sinne der vorliegenden Erfindung auch als osteokonduktive Leitstruktur bzw. osteokonduktive Leitmatrix bezeichnet werden.
   Die Hohl- bzw. Zwischenräume der Struktur bzw. Matrix können einen Durchmesser, von 0,1 mm bis 1,2 mm, insbesondere 0,2 mm bis 1 mm, bevorzugt 0,3 mm bis 0,8 mm, aufweisen.
- Im Gegensatz zu gattungsgemäßen Implantaten, insbesondere metallischen Augmentaten, bei welchen lediglich Zwischenräume an Implantatrandzonen mechanisch belastet werden, können in einem verdichteten Zustand der osteokonduktiven Stützkörper vorhandene Hohl- und/oder Zwischenräume aufgrund eines geringen E-Moduls auch innerhalb einer kompletten Defektauffüllung, d.h. homogen, mechanisch belastet werden (Mikrobewegungen). Dies wiederum bewirkt eine Stimulierung und/oder Verstärkung von Knochenwachstum, insbesondere von Knochenneubildung. Insgesamt ist dadurch eine homogene Verknöcherung des gesamten Knochendefekts erzielbar.

In Ausgestaltung der Erfindung beruht die Löslichkeit des Umhüllungsmaterials auf einem physikalischen Lösungsvorgang, d.h. das Umhüllungsmaterial ist ohne Abbau oder sonstige Zerstörung in Wasser oder einer wasserhaltigen Flüssigkeit löslich.

In weiterer Ausgestaltung der Erfindung beruht die Löslichkeit des Umhüllungsmaterials auf einem Abbau, insbesondere auf einer Hydrolyse, wie beispielsweise einer enzymkatalysierten Hydrolyse, des löslichen Umhüllungsmaterials.

In weiterer Ausgestaltung der Erfindung ist das lösliche Umhüllungsmaterial während eines Zeitraums von 1 Sekunde bis 72 Stunden, insbesondere 1 Minute bis 1 Stunde, bevorzugt 2 Minuten bis 20 Minuten, in Wasser oder einer wasserhaltigen Flüssigkeit, insbesondere Körperflüssigkeit, löslich.

In weiterer Ausgestaltung der Erfindung weist das lösliche Umhüllungsmaterial ein Polysaccharid auf oder besteht aus einem Polysaccharid. Bei dem Polysaccharid kann es sich insbesondere um ein Mucopolysaccharid und/oder ein Cellulosederivat, insbesondere eine Alkylcellulose und/oder Hydroxyalkylcellulose, handeln. Vorzugsweise ist das Polysaccharid ausgewählt aus der Gruppe bestehend aus Stärke, Amylose, Amylopektin, Dextran, Methylcellulose, Hydroxymethylcellulose, Ethylcellulose, Hydroxyethylcellulose, Propylcellulose, Hydroxypropylcellulose, Butylcellulose, Hydroxybutylcellulose, Hydroxyethylmethylcellulose, Hydroxypropylmethylcellulose, Carboxymethylcellulose, Hyaluronsäure, Chondroitin-4-sulfat, Chondroitin-6-sulfat, Keratinsulfat, Algininsäure, Heparin, Heparansulfat, Chitosan, Salze davon oder Mischungen davon.

In weiterer Ausgestaltung der Erfindung weist das lösliche Umhüllungsmaterial ein synthetisches Polymer auf oder besteht aus einem solchen Polymer. Bevorzugt ist das synthetische Polymer ausgewählt aus der Gruppe bestehend aus Polyvinylalkohol, Polyethylenglykol, Ethylenoxid-Propylenoxid-Copolymere (EO-PO-Copolymere), Ethylenoxid-Propylenoxid-Blockcopolymere (EO-PO-Blockcopolymere), Acrylsäure-Homopolymere, Acrylsäure-Copolymere, Polyvinylpyrrolidon-Homopolymere, Polyvinylpyrrolidon-Copolymere oder Mischungen davon.

Bei dem im vorherigen Absatz genannten Polyvinylalkohol kann es sich insbesondere um einen sogenannten niedermolekularen Polyvinylalkohol, d.h. um einen Polyvinylalkohol mit einem mittleren Molekulargewicht von 10.000 bis 30.000, und/oder um einen sogenannten hochmolekularen Polyvinylalkohol, d.h. um einen Polyvinylalkohol mit einem mittleren Molekulargewicht von 50.000 bis 250.000, handeln.

In weiterer Ausgestaltung der Erfindung weist das lösliche Umhüllungsmaterial einen niedermolekularen Polyvinylalkohol, d.h. einen Polyvinylalkohol mit einem mittleren Molekulargewicht von 10.000 bis 30.000, und/oder einen hochmolekularen Polyvinylalkohol, d.h. einen Polyvinylalkohol mit einem mittleren Molekulargewicht von 50.000 bis 250.000, sowie ferner ein Polysaccharid, insbesondere eine Carboxyalkylcellulose, vorzugsweise Carboxymethylcellulose, auf.

In weiterer Ausgestaltung der Erfindung weist das lösliche Umhüllungsmaterial eine Mischung aus einem Polysaccharid und einem synthetischen Polymer auf oder besteht aus einer solchen Mischung. Bezüglich eines geeigneten Polysaccharids sowie synthetischen Polymers wird auf die in den vorherigen Absätzen beschriebenen Polysaccharide sowie synthetischen Polymere Bezug genommen.

Das lösliche Umhüllungsmaterial kann weiterhin einen Anteil von 0,1 Gew.-% bis 100 Gew.-%, insbesondere 5 Gew.-% bis 100 Gew.-%, vorzugsweise 90 Gew.-% bis 100 Gew.-%, aufweisen, bezogen auf das Gesamtgewicht der Umhüllung. Je höher der Anteil des löslichen Umhüllungsmaterials ist, desto vollständiger kann die Umhüllung in Wasser oder einer wasserhaltigen Flüssigkeit aufgelöst werden. Somit lässt sich über den Anteil des löslichen Umhüllungsmaterials mit besonderem Vorteil die Löslichkeit der Umhüllung steuern.

In weiterer Ausgestaltung der Erfindung ist das lösliche Umhüllungsmaterial wenigstens teilweise, insbesondere nur teilweise oder vollständig, vernetzt. Das lösliche Umhüllungsmaterial kann chemisch und/oder physikalisch vernetzt sein. Beispielsweise kann das Umhüllungsmaterial mittels eines chemischen Vernetzungsmittels, insbesondere ausgewählt aus der Gruppe bestehend aus Formaldehyd, Dialdehyd wie Glutaraldehyd, Polyaldehyde, Diisocyanate, Dicarbodiimide oder Mischungen davon, vernetzt sein. Das chemische Vernetzungsmittel kann dabei einen Anteil von 0,1 Gew.-% bis 20 Gew.-%, insbesondere 1 Gew.-% bis 10 Gew.-%, vorzugsweise 2 Gew.-% bis 5 Gew.-%, aufweisen, bezogen auf das Gesamtgewicht der Umhüllung. Alternativ oder in Kombination kann das Umhüllungsmaterial mittels Bestrahlung, insbesondere Gamma-Bestrahlung, und/oder mittels Gefrier-Tau-Zyklen, vernetzt sein. Über eine Vernetzung, insbesondere über die Art und/oder den Grad einer Vernetzung, oder über eine fehlende Vernetzung lässt sich ebenfalls mit besonderem Vorteil die Löslichkeit des Umhüllungsmaterials und mithin der Umhüllung in Wasser oder einer wasserhaltigen Flüssigkeit steuern, wobei eine Vernetzung generell eine Verringerung der Löslichkeit bzw. eine Erhöhung der Lösungsdauer bewirkt.

In weiterer Ausgestaltung der Erfindung ist die Umhüllung frei von einem Vernetzungsmittel.

In weiterer Ausgestaltung der Erfindung weist die Umhüllung ferner ein in Wasser oder einer wasserhaltigen Flüssigkeit unlösliches Umhüllungsmaterial auf. Ein solches Umhüllungsmaterial wird nachfolgend auch als unlösliches Umhüllungsmaterial abgekürzt.

Das unlösliche Umhüllungsmaterial ist vorzugsweise ein Polymer, insbesondere ein hydrophobes Polymer.

Bevorzugt ist das Polymer ausgewählt aus der Gruppe bestehend aus Polyolefin, Polyester, Polyamid, Polyurethan wie thermoplastisches Polyurethan, Elastomer, Polyimid, Polyetherketon, Polyetheretherketon, Fluorcarbon, Perfluorcarbon oder Mischungen davon.

Das Polyolefin kann ausgewählt sein aus der Gruppe bestehend aus Polyethylen, Polypropylen, Polyacrylat, Polymethacrylat, Polymethylmethacrylat, Polyvinylidenchlorid, Polivinylidenfluorid, Polytetrafluorethylen, Polytetrafluorpropylen, Polyhexafluorpropylen, Copolymere davon oder Mischungen davon.

Bei dem Polyethylen kann es sich insbesondere um Polyethylen niedriger Dichte, Polyethylen mittlerer Dichte, Polyethylen hoher Dichte, ultrahochmolekulares Polyethylen, ein Copolymer davon oder Mischungen davon.Der Polyester kann insbesondere ausgewählt sein aus der Gruppe bestehend aus Polyethylenterephthalat, Polypropylenterephthalat, Polybutylenterephthalat, Copolymere davon oder Mischungen davon.

Das Polyamid kann insbesondere ausgewählt sein aus der Gruppe bestehend aus Polyamid 6 (Polyamid aus Caprolactam), Polyamid 46 (Polyamid aus Tetramethylendiamin und Adipinsäure), Polyamid 6.6 (Polyamid aus Hexamethylendiamin und Adipinsäure), Polyamid 69 (Polyamid aus Hexamethylendiamin und Azelainsäure), Polyamid 6/12 (Polyamid aus Hexamethylendiamin und Dodecandisäure), Polyamid 1010 (Polyamid aus 1,10-Decandiamin und Sebacinsäure), Polyamid 11 (Polyamid aus alpha-Aminoundecansäure), Polyamid 12 (Polyamid aus Laurinlactam), Polyamid 1212 (Polyamid aus Dodecandiamin und Dodecandisäure), Seide, Copolymere davon oder Mischungen davon.

Das unlösliche Umhüllungsmaterial kann einen Anteil von 0,1 Gew.-% bis 99 Gew.-%, insbesondere 0,1 Gew.-% bis 50 Gew.-%, vorzugsweise 0,1 Gew.-% bis 1 Gew.-%, aufweisen, bezogen auf das Gesamtgewicht der Umhüllung. Auch über den Anteil eines optional vorhandenen unlöslichen Umhüllungsmaterials kann die Löslichkeit der Umhüllung bewusst beeinflusst werden. Dabei verringert ein Anteil eines unlöslichen Umhüllungsmaterials insgesamt die Löslichkeit der Umhüllung.

Weiterhin kann das unlösliche Umhüllungsmaterial insbesondere in Form eines Additivs der Umhüllung vorliegen.

Unter dem Ausdruck "Additiv" soll im Sinne der vorliegenden Erfindung im Zusammenhang der Umhüllung ein Zusatzstoff verstanden werden, welcher einen maximalen Anteil von 49 Gew.-%, insbesondere von 0,1 bis 40 Gew.-%, bevorzugt 2 Gew.-% bis 20 Gew.-%, aufweist, bezogen auf das Gesamtgewicht der Umhüllung.

In weiterer Ausgestaltung der Erfindung ist die Umhüllung frei von einem in Wasser oder einer wasserhaltigen Flüssigkeit unlöslichen Umhüllungsmaterial.

In weiterer Ausgestaltung der Erfindung weist die Umhüllung oder ein Teilbereich der Umhüllung eine Dicke von 10 µm bis 1 mm, insbesondere 20 µm bis 800 µm, vorzugsweise 30 µm bis 300 µm, auf. Die Umhüllung kann dabei insbesondere eine einheitliche Dicke oder, wie im Folgenden noch näher erläutert werden wird, eine uneinheitliche Dicke, d.h. eine ungleichförmig verlaufende Dicke, aufweisen.

In weiterer Ausgestaltung der Erfindung ist die Umhüllung oder ein Teilbereich der Umhüllung folienförmig gestaltet. Mit anderen Worten liegt die Umhüllung oder ein Teilbereich davon in weiterer Ausgestaltung der Erfindung in Form einer Folie vor. Ein folienförmiger Teilbereich der Umhüllung weist vorzugsweise ein in Wasser oder einer wasserhaltigen Flüssigkeit lösliches Umhüllungsmaterial auf oder besteht aus einem solchen Umhüllungsmaterial. Weiterhin ist es bevorzugt, wenn ein folienförmiger Teilbereich der Umhüllung im implantierten Zustand knochenzugewandt angeordnet ist. Bezüglich geeigneter löslicher Umhüllungsmaterialien sei auf die bisherige Beschreibung verwiesen.

Der im Zusammenhang der Umhüllung verwendete Ausdruck "folienförmig" oder "Folie" soll im Sinne der vorliegenden Erfindung eine Umhüllung definieren, welche vorzugsweise eine Dicke knochenzugewandt angeordnet ist. Bezüglich geeigneter löslicher Umhüllungsmaterialien sei auf die bisherige Beschreibung verwiesen.

Der im Zusammenhang der Umhüllung verwendete Ausdruck "folienförmig" oder "Folie" soll im Sinne der vorliegenden Erfindung eine Umhüllung definieren, welche vorzugsweise eine Dicke von 10 µm bis 1 mm, insbesondere 20 µm bis 800 µm, vorzugsweise 30 µm bis 300 µm, aufweist. Eine derartig dünne Ausgestaltung der Umhüllung ist besonders vorteilhaft im Hinblick auf eine möglichst schnelle Löslichkeit des Umhüllungsmaterials und somit der Umhüllung.

In weiterer Ausgestaltung der Erfindung ist ein Teilbereich der Umhüllung textilförmig, insbesondere netzförmig, d.h. in Form eines Netzes, gestaltet. Vorzugsweise weist der Teilbereich ein in Wasser oder einer wasserhaltigen Flüssigkeit unlösliches Umhüllungsmaterial auf oder besteht aus einem solchen Umhüllungsmaterial. Weiterhin ist es bevorzugt, wenn der Teilbereich im implantierten Zustand einer künstlichen Gelenkpfanne, insbesondere einer künstlichen Hüftgelenkpfanne, zugewandt angeordnet ist. Bezüglich geeigneter unlöslicher Umhüllungsmaterialien sei auf die bisherige Beschreibung verwiesen.

Unter dem Ausdruck "netzförmig" oder "Netz" soll im Sinne der vorliegenden Erfindung die Form eines textilen Flächengebildes bzw. ein textiles Flächengebilde verstanden werden, welches vorzugsweise regelmäßige Maschen oder Öffnungen aufweist, wobei die Maschen bzw. Öffnungen beispielsweise rhombisch, quadratisch oder sechseckig gestaltet sein können. Insbesondere soll unter dem Ausdruck "netzförmig" oder "Netz" die Form eines gewirkten oder gestrickten Flächengebildes bzw. ein gewirktes oder gestricktes Flächengebilde verstanden werden.

In weiterer Ausgestaltung der Erfindung weist die Umhüllung als Teilbereiche, welche unterschiedlich gestaltet sind, einen ersten Teilbereich und einen zweiten Teilbereich. Dadurch ist es insbesondere möglich, eine richtungs- und/oder seitenabhängige Löslichkeit der Umhüllung in Wasser oder einer wasserhaltigen Flüssigkeit zu realisieren.

In weiterer Ausgestaltung der Erfindung unterscheiden sich die Teilbereiche voneinander in Bezug auf die Löslichkeit der Umhüllung in Wasser oder einer wasserhaltigen Flüssigkeit und/oder in Bezug auf die Dicke der Umhüllung und/oder in Bezug auf die Struktur der Umhüllung.

In weiterer Ausgestaltung der Erfindung unterscheiden sich die Teilbereiche voneinander in Bezug auf die chemische Zusammensetzung, insbesondere in Bezug auf eine Vernetzung, des Insbesondere können sich die Teilbereiche in Bezug auf die Art einer Vernetzung und/oder den Gad einer Vernetzung voneinander unterscheiden. Beispielsweise können sich die Teilbereiche in Bezug auf ein chemisches Vernetzungsmittel und/oder eine physikalische Vernetzung voneinander unterscheiden. Bezüglich geeigneter chemischer Vernetzungsmittel sowie physikalischer Vernetzungen sei auf die vorherigen im Zusammenhang einer optionalen Vernetzung des löslichen Umhüllungsmaterials gemachten Ausführungen verwiesen.

In weiterer Ausgestaltung der Erfindung weist der erste Teilbereich ein besser oder schneller in Wasser oder einer wasserhaltigen Flüssigkeit lösliches Umhüllungsmaterial auf oder besteht aus einem solchen Umhüllungsmaterial als der zweite Teilbereich. Vorzugsweise ist der erste Teilbereich im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist. Dadurch kann in besonders vorteilhafter Weise erreicht werden, dass sich die Umhüllung zunächst nur in dem Bereich auflöst, bezüglich dessen mit einem Einwachsen von Knochengewebe gerechnet werden kann, wohingegen sich der restliche Teil der Umhüllung durch eine vergleichsweise höhere Beständigkeit gegenüber Wasser oder einer wasserhaltigen Flüssigkeit auszeichnet, so dass wenigstens während einer Anfangsphase nach Implantation eine Teilintegrität der Umhüllung aufrechterhalten werden kann. Bezüglich geeigneter Umhüllungsmaterialien wird auf die bisherige Beschreibung Bezug genommen.

In weiterer Ausgestaltung der Erfindung weist der erste Teilbereich ein schwächer vernetztes, in Wasser oder einer wasserhaltigen Flüssigkeit lösliches Umhüllungsmaterial auf oder besteht aus einem solchen Umhüllungsmaterial als der zweite Teilbereich. Mit anderen Worten weist in weiterer Ausgestaltung der Erfindung der erste Teilbereich ein in Wasser oder einer wasserhaltigen Flüssigkeit lösliches Umhüllungsmaterial mit einem niedrigeren Vernetzungsgrad auf oder besteht aus einem solchen Umhüllungsmaterial als der zweite Teilbereich. Vorzugsweise ist der erste Teilbereich im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist. Bezüglich geeigneter Umhüllungsmaterialien wird auf die bisherige Beschreibung Bezug genommen.

In weiterer Ausgestaltung der Erfindung weist der erste Teilbereich ein nicht vernetztes, in Wasser oder einer wasserhaltigen Flüssigkeit lösliches Umhüllungsmaterial auf oder besteht aus einem solchen Umhüllungsmaterial, wohingegen der zweite Teilbereich ein vernetztes, in Wasser oder einer wasserhaltigen Flüssigkeit lösliches Umhüllungsmaterial aufweist oder aus einem solchen Umhüllungsmaterial besteht. Vorzugsweise ist der erste Teilbereich im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist. Bezüglich geeigneter Umhüllungsmaterialien wird auf die bisherige Beschreibung Bezug genommen.

In weiterer Ausgestaltung der Erfindung weist der erste Teilbereich einen höheren Anteil eines in Wasser oder einer wasserhaltigen Flüssigkeit löslichen Umhüllungsmaterials auf als der zweite Teilbereich. Vorzugsweise ist der erste Teilbereich im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist. Bezüglich geeigneter Umhüllungsmaterialien wird auf die bisherige Beschreibung Bezug genommen.

In weiterer Ausgestaltung der Erfindung weist der erste Teilbereich ein in Wasser oder einer wasserhaltigen Flüssigkeit lösliches Umhüllungsmaterial auf oder besteht aus einem solchen Umhüllungsmaterial, wohingegen der zweite Teilbereich ein in Wasser oder einer wasserhaltigen Flüssigkeit unlösliches Umhüllungsmaterial aufweist oder aus einem solchen Umhüllungsmaterial besteht. Vorzugsweise ist der erste Teilbereich im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist. Bezüglich geeigneter Umhüllungsmaterialien wird auf die bisherige Beschreibung Bezug genommen.

In weiterer Ausgestaltung der Erfindung weist der erste Teilbereich eine kleinere Dicke auf als der zweite Teilbereich. Vorzugsweise ist der erste Teilbereich im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist.

In weiterer Ausgestaltung der Erfindung ist der erste Teilbereich nicht textilförmig, insbesondere folienförmig, und der zweite Teilbereich textilförmig, insbesondere netzförmig, d.h. in Form eines Netzes, gestaltet. Vorzugsweise ist der erste Teilbereich dabei im implantierten Zustand knochenzugewandt angeordnet, wohingegen der zweite Teilbereich der Umhüllung im implantierten Zustand einer künstlichen Gelenkpfanne, insbesondere einer künstlichen Hüftgelenkpfanne, zugewandt angeordnet ist.

In weiterer Ausgestaltung der Erfindung ist die Umhüllung formstabil, insbesondere an einen Knochendefekt angepasst, gestaltet.

In weiterer Ausgestaltung der Erfindung ist die Umhüllung nur teilweise mit den osteokonduktiven Stützkörpern befüllt. Beispielsweise kann ein durch die Umhüllung definiertes Hohlraumvolumen lediglich zu 50% bis 80% mit den osteokonduktiven Stützkörpern befüllt sein. Auf diese Weise ist mit besonderem Vorteil eine formbare und mithin an den zu behandelnden und/oder biologisch zu rekonstruierenden Knochendefekt anpassbare Umhüllung realisierbar.

In weiterer Ausgestaltung der Erfindung handelt es sich bei den osteokonduktiven Stützkörpern um vereinzelte osteokonduktive Stützkörper. Mit anderen Worten liegen die osteokonduktiven Stützkörper in weiterer Ausgestaltung der Erfindung in vereinzelter Form vor, d.h. in Form von einzelnen Stützkörpern, beispielsweise in Form eines Schüttguts, wie im Folgenden noch näher erläutert werden wird.

In weiterer Ausgestaltung der Erfindung weisen die osteokonduktiven Stützkörper Apatit und/oder Tricalciumphosphat auf oder bestehen aus Apatit und/oder Tricalciumphosphat. Die osteokonduktiven Stützkörper können jeweils einen Anteil an Apatit von 0,1 Gew.-% bis 100 Gew.-% aufweisen. Weiterhin können die osteokonduktiven Stützkörper jeweils einen Anteil an Tricalciumphosphat von 0,1 Gew.-% bis 100 Gew.-% aufweisen.

Vorzugsweise handelt es sich bei dem Apatit um einen nicht in vivo abbaubaren oder um einen nicht in vivo resorbierbaren Apatit. Dadurch kann selbst bei Patienten, bei welchen nicht mehr mit einem (ausreichenden) Knochenwachstum zu rechnen ist, eine ausreichende Sekundärstabilität erzielt werden. Dies ist insbesondere im Hinblick auf die Behandlung von Knochendefekten älterer Patienten von Vorteil.

Alternativ kann es sich bei dem Apatit um einen in vivo abbaubaren oder um einen in vivo resorbierbaren Apatit, vorzugsweise um einen langsam in vivo abbaubaren oder um einen langsam in vivo resorbierbaren Apatit, handeln. Insbesondere kann der Apatit eine in vivo Abbauzeit (Degradationszeit) oder eine in vivo Resorptionszeit von 6 Monaten bis 30 Jahre, insbesondere 1 Jahr bis 20 Jahre, bevorzugt 4 Jahren bis 10 Jahre, aufweisen. Die in diesem Absatz offenbarten Abbau- bzw. Resorptionszeiten sind insbesondere im Hinblick auf die Behandlung von Patienten mit einem langsamen oder gar fehlenden Knochenwachstum von Vorteil.

Der Apatit kann weiterhin insbesondere in kristalliner Form vorliegen. Durch eine hohe Kristallinität kann eine hohe Festigkeit erreicht werden. Durch eine niedrige Kristallinität kann eine gute und/oder schnelle Abbaubarkeit erreicht werden.

Weiterhin kann es sich bei dem Apatit um einen mikrokristallinen Apatit, d.h. um einen Apatit mit Kristalliten, welche wenigstens eine Abmessung oder Dimension im Mikrometerbereich, insbesondere in einem Bereich von > 0,5 µm, insbesondere 0,6 µm bis 500 µm, bevorzugt 0,6 µm bis 100 µm, aufweisen, handeln. Bei der wenigstens einen Abmessung bzw. Dimension kann es sich um die Länge und/oder Breite (Dicke bzw. Höhe) und/oder den Durchmesser, insbesondere mittleren Durchmesser, der Kristallite handeln.

Grundsätzlich kann es sich bei dem Apatit aber auch um einen makrokristallinen Apatit handeln.

Weiterhin kann es sich bei dem Apatit um einen nanokristallinen Apatit, d.h. um einen Apatit mit Kristalliten, welche wenigstens eine Abmessung oder Dimension im Nanometerbereich, insbesondere in einem Bereich von 0,1 nm bis 500 nm, bevorzugt 0,1 nm bis 100 nm, aufweisen, handeln. Bei der wenigstens einen Abmessung bzw. Dimension kann es sich um die Länge und/oder Breite (Dicke bzw. Höhe) und/oder den Durchmesser, insbesondere mittleren Durchmesser, der Kristallite handeln.

Weiterhin kann der Apatit in amorpher Form vorliegen. Dadurch kann eine besonders gute und/oder schnelle Resorption erreicht werden.

Weiterhin kann es sich bei dem Apatit um einen phasenreinen Apatit handeln. Unter dem Ausdruck "phasenrein" soll insbesondere phasenrein im Sinne einer einschlägigen Vorschrift, vorzugsweise nach ASTM F1185, verstanden werden.

Weiterhin kann der Apatit eine Porosität kleiner 50 %, insbesondere kleiner 20 %, vorzugsweise kleiner 15 %, aufweisen. Durch eine geringe Porosität kann eine hohe mechanische Stabilität erreichen werden.

Weiterhin kann der Apatit nicht porös ausgebildet sein.

Weiterhin können die osteokonduktiven Stützkörper eine Mischung aus porösen Apatiten und/oder Apatiten mit unterschiedlicher Porosität und/oder nichtporösen Apatiten aufweisen oder aus einer solchen Mischung bestehen, insbesondere wenn die Stützkörper nach einem additiven Fertigungsverfahren hergestellt sind.

Weiterhin kann es sich bei dem Apatit um natürlich vorkommenden Apatit oder um einen aus natürlichem Apatit gewonnenen Apatit handeln.

Weiterhin kann es sich bei dem Apatit um einen synthetischen, d.h. künstlichen oder artifiziellen, Apatit handeln.

Bevorzugt ist der Apatit ausgewählt aus der Gruppe bestehend aus Hydroxylapatit, Fluorapatit, Chlorapatit, Carbonat-Fluor-Apatit oder Mischungen davon.

Besonders bevorzugt handelt es sich bei dem Apatit um Hydroxylapatit. Beispielsweise kann es sich bei dem Hydroxylapatit um einen vollsynthetischen, nanokristallinen und phasenreinen Hydroxylapatit handeln. Ein solcher Hydroxylapatit ist im Handel beispielsweise unter der Bezeichnung Ostim kommerziell erhältlich.

Der Apatit und das Tricalciumphoshat kann auch als biphasische Mischung vorliegen. Die biphasische Struktur ist von Vorteil, da die Knochenzellen einwachsen können und das Material so sukzessive ersetzt wird. Dabei weist der Apatit und/oder das Tricalciumphosphat und/oder das biphasische Calciumphosphat eine Porosität von 1 % bis 50 %, insbesondere 5 % bis 20 %, bevorzugt 10 % bis 15 % auf.

Weiterhin kann es sich bei dem Apatit um einen gesinterten Apatit handeln.

Vorzugsweise ist der gesinterte Apatit ausgewählt aus der Gruppe bestehend aus gesinterter Hydroxylapatit, gesinterter Fluorapatit, gesinterter Chlorapatit, gesinterter Carbonat-Fluor-Apatit oder Mischungen davon.

In weiterer Ausgestaltung der Erfindung weisen die osteokonduktiven Stützkörper Tricalciumphosphat auf oder bestehen aus Tricalciumphosphat.

Vorzugsweise handelt es sich bei dem Tricalciumphosphat um ein nicht in vivo abbaubares oder um ein nicht in vivo resorbierbares Tricalciumphosphat. Dadurch kann selbst bei Patienten, bei welchen nicht mehr mit einem (ausreichenden) Knochenwachstum zu rechnen ist, eine ausreichende Sekundärstabilität erzielt werden. Dies ist insbesondere im Hinblick auf die Behandlung von Knochendefekten älterer Patienten von Vorteil.

Alternativ kann es sich bei dem Tricalciumphosphat um ein in vivo abbaubares oder um ein in vivo resorbierbares Tricalciumphosphat, vorzugsweise um ein langsam in vivo abbaubares oder um ein langsam in vivo resorbierbares Tricalciumphosphat, handeln. Insbesondere kann das Tricalciumphosphat eine in vivo Abbauzeit (Degradationszeit) oder eine in vivo Resorptionszeit von 1 Monat bis 15 Jahre, insbesondere 6 Monaten bis 10 Jahre, bevorzugt 1 Jahr bis 5 Jahre, aufweisen. Die in diesem Absatz offenbarten Abbau- bzw. Resorptionszeiten sind insbesondere im Hinblick auf die Behandlung von Patienten mit einem langsamen Knochenwachstum von Vorteil.

Das Tricalciumphosphat kann weiterhin in kristalliner Form vorliegen. Durch eine hohe Kristallinität kann eine hohe Festigkeit erreicht werden. Durch eine niedrige Kristallinität kann eine gute und/oder schnelle Abbaubarkeit erreicht werden. Bevorzugt weist das Tricalciumphosphat eine Kristallinität von 50 % bis 99 %, insbesondere 75 % bis 95 %, auf.

Weiterhin kann es sich bei dem Tricalciumphosphat um mikrokristallines Tricalciumphosphat, d.h. um Tricalciumphosphat mit Kristalliten, welche wenigstens eine Abmessung oder Dimension im Mikrometerbereich, insbesondere in einem Bereich von > 0,5 µm, insbesondere 0,6 µm bis 500 µm, bevorzugt 0,6 µm bis 100 µm, aufweisen, handeln. Bei der wenigstens einen Abmessung bzw. Dimension kann es sich um die Länge und/oder Breite (Dicke bzw. Höhe) und/oder den Durchmesser, insbesondere mittleren Durchmesser, der Kristallite handeln.

Grundsätzlich kann es sich bei dem Tricalciumphosphat aber auch um ein makrokristallines Tricalciumphosphat handeln.

Weiterhin kann es sich bei dem Tricalciumphosphat um nanokristallines Tricalciumphosphat, d.h. um Tricalciumphosphat mit Kristalliten, welche wenigstens eine Abmessung oder Dimension im Nanometerbereich, insbesondere in einem Bereich von 0,1 nm bis 500 nm, bevorzugt 0,1 nm bis 100 nm, aufweisen, handeln. Bei der wenigstens einen Abmessung bzw. Dimension kann es sich um die Länge und/oder Breite (Dicke bzw. Höhe) und/oder den Durchmesser, insbesondere mittleren Durchmesser, der Kristallite handeln.

Weiterhin kann das Tricalciumphosphat in amorpher Form vorliegen. Dadurch kann eine besonders gute und/oder schnelle Resorption erreicht werden.

Weiterhin kann es sich bei dem Tricalciumphosphat um ein phasenreines Tricalciumphosphat handeln. Unter dem Ausdruck "phasenrein" soll insbesondere phasenrein im Sinne einer einschlägigen Vorschrift, vorzugsweise nach ASTM F1088, verstanden werden.

Weiterhin kann das Tricalciumphosphat eine Porosität kleiner 50 %, insbesondere kleiner 20 %, vorzugsweise kleiner 15 %, aufweisen.

Weiterhin kann das Tricalciumphosphat nicht porös ausgebildet sein.

Weiterhin kann es sich bei dem Tricalciumphosphat um natürlich vorkommendes Tricalciumphosphat oder um ein aus natürlichem Tricalciumphosphat gewonnenes Tricalciumphosphat handeln.

Weiterhin kann es sich bei dem Tricalciumphosphat um ein synthetisches, d.h. künstliches oder artifizielles, Tricalciumphosphat handeln.

Das Tricalciumphosphat ist bevorzugt ausgewählt aus der Gruppe bestehend aus alpha-Tricalciumphosphat (α-TCP), beta-Tricalciumphosphat (β-TCP) und einer Mischung aus alpha-Tricalciumphosphat und beta-Tricalciumphosphat.

Insbesondere kann es sich bei dem Tricalciumphosphat um gesintertes Tricalciumphosphat handeln.

Das gesinterte Tricalciumphosphat ist vorzugsweise ausgewählt aus der Gruppe bestehend aus gesintertes alpha-Tricalciumphosphat, gesintertes beta-Tricalciumphosphat und einer Mischung aus gesintertem alpha-Tricalciumphosphat und gesintertem beta-Tricalciumphosphat.

Weiterhin können die osteokonduktiven Stützkörper Apatit und Tricalciumphosphat aufweisen oder aus Apatit und Tricalciumphosphat bestehen. Vorzugsweise weisen die osteokonduktiven Stützkörper Hydroxylapatit und beta-Tricalciumphosphat auf oder bestehen aus Hydroxylapatit und beta-Tricalciumphosphat. Bezüglich weiterer Merkmale und Vorteile des Apatits sowie des Tricalciumphosphats wird auf die vorangegangenen Ausführungen Bezug genommen.

In weiterer Ausgestaltung der Erfindung weisen die osteokonduktiven Stützkörper eine angeraute Oberfläche auf. Dadurch kann ein Anwachsen oder Anhaften von Knochengewebe, insbesondere an eine durch die Stützkörper gebildete osteokonduktive Leitstruktur, optimiert werden. Unter dem Ausdruck "anrauen" soll im Sinne der vorliegenden Erfindung insbesondere verstanden werden, dass eine Rauheit der Oberfläche nach einer Ausformung der Stützkörper erhöht wird, insbesondere in einem dafür vorgesehenen Herstellungsschritt. Das Anrauen kann zum Beispiel durch eine Anätzung, insbesondere mittels Phosphorsäure, geschehen. Vorzugsweise weisen die Stützkörper eine angeraute Oberfläche auf, deren Rauigkeit gegenüber einer nicht angerauten Stützkörperoberfläche um wenigstens 10 % erhöht ist. Unter dem Ausdruck "Rauheit" soll insbesondere eine Unebenheit der Oberfläche der osteokonduktiven Stützkörper verstanden werden.

In einem weiteren Beispiel sind die Stützkörper mittels eines additiven Fertigungsverfahrens hergestellt.

In weiterer Ausgestaltung der Erfindung weisen die Stützkörper Calciumphosphatzement auf oder bestehen aus Calciumphosphatzement. Bei dem Calciumphosphatzement kann es sich insbesondere um einen Calciumphosphatzement handeln, welcher vor einem vollständigen Aushärten einem Druck, vorzugsweise absoluten Druck, von wenigstens 2 bar unterworfen wird. Auf diese Weise kann mit besonderem Vorteil die Porosität erniedrigt werden.

In weiterer Ausgestaltung der Erfindung sind die osteokonduktiven Stützkörper stoffschlüssig miteinander verbunden, insbesondere miteinander verklebt.

In weiterer Ausgestaltung der Erfindung sind die osteokonduktiven Stützkörper mit einem Bindemittel beschichtet. Bei dem Bindemittel handelt es sich vorzugsweise um ein Bindemittel, welches durch Hitze oder ein Lösungsmittel, wie beispielsweise N-Methyl-Pyrrolidon (NMP), angelöst werden kann. Durch die Verwendung eines derartigen Bindemittels ist es möglich, die osteokonduktiven Stützkörper durch Erhitzen und anschließendes Abkühlen oder durch Zugabe eines Lösungsmittels miteinander zu verbinden. Bei dem Bindemittel kann es sich beispielsweise um Polylactid und/oder Poly(lactid-co-Glycolid) (PLGA) handeln.

In weiterer Ausgestaltung der Erfindung sind die osteokonduktiven Stützkörper derart ausgebildet, das sie ein Verdichten, insbesondere Impaktieren, der Stützkörper, beispielsweise mittels eines geeigneten Instruments wie eines Nachschlägers, begünstigen. Bezüglich entsprechend geeigneter Ausgestaltungen der osteokonduktiven Stützkörper sei auf die nachfolgenden Ausführungen verwiesen.

In weiterer Ausgestaltung der Erfindung sind die osteokonduktiven Stützkörper regelmäßig geformt, d.h. liegen als Formkörper vor. Unter dem Ausdruck "regelmäßig geformt" oder "Formkörper" sollen im Sinne der vorliegenden Erfindung insbesondere die nachfolgend beschriebenen Formen verstanden werden.

Die osteokonduktiven Stützkörper, insbesondere Formkörper, können einen polygonen Querschnitt aufweisen. Beispielsweise können die osteokonduktiven Stützkörper, insbesondere Formkörper, einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt aufweisen.

Weiterhin können die osteokonduktiven Stützkörper, insbesondere Formkörper, unterschiedliche Querschnitte aufweisen. Bezüglich möglicher Querschnitte wird auf die im vorherigen Absatz genannten Querschnitte Bezug genommen.

Die osteokonduktiven Stützkörper, insbesondere Formkörper, können weiterhin polyederförmig, insbesondere quader-, würfel-, tetraeder-, prismen-, pyramiden-, pyramidenstumpf- oder spatförmig, ausgebildet sein.

Weiterhin können die osteokonduktiven Stützkörper, insbesondere Formkörper, unterschiedlich polyederförmig ausgebildet sein. Mit anderen Worten können die osteokonduktiven Stützkörper, insbesondere Formkörper, in unterschiedlichen Polyederformen vorliegen. Bezüglich möglicher Polyederformen wird auf den vorherigen Absatz Bezug genommen.

Weiterhin können die osteokonduktiven Stützkörper, insbesondere Formkörper, einen eckenlosen, Querschnitt aufweisen. Beispielsweise können die Strukturelemente einen ovalförmigen, insbesondere kreisförmigen oder elliptischen, Querschnitt aufweisen.

Weiterhin können die osteokonduktiven Stützkörper, insbesondere Formkörper, nichtpolyederförmig, insbesondere kugel-, kegel-, kegelstumpf-, ring-, toroid- oder kreiszylinderförmig, ausgebildet sein.

Weiterhin können die osteokonduktiven Stützkörper, insbesondere Formkörper, unterschiedlich nichtpolyederförmig ausgebildet sein. Mit anderen Worten können die osteokonduktiven Stützkörper, insbesondere Formkörper, in unterschiedlichen Nichtpolyederformen vorliegen. Bezüglich möglicher Nichtpolyederformen wird auf den vorherigen Absatz Bezug genommen.

Weiterhin können die osteokonduktiven Stützkörper, insbesondere Formkörper, in Form von Oligopoden, d.h. oligopodenförmig, gestaltet sein.

Die Oligopoden können insbesondere konusförmig und insbesondere rotationssymmetrisch gestaltete Beine aufweisen. Die Beine können einen Konuswinkel von 5° bis 25°, insbesondere 7° bis 15°, aufweisen.

Weiterhin können die Oligopoden Beine mit einer Länge von 0,5 mm bis 5 mm, insbesondere 1,5 mm bis 2,5 mm, aufweisen.

Des Weiteren können die Oligopoden Beine mit einem mittleren Durchmesser von 0,2 mm bis 3 mm, insbesondere 0,3 mm bis 0,7 mm, aufweisen.

Die Oligopoden können weiterhin ausgewählt sein aus der Gruppe bestehend aus Tripoden, Tetrapoden, Pentapoden, Hexapoden, Heptapoden, Oktapoden oder Mischungen davon.

Erfindungsgemäß kann es insbesondere bevorzugt sein, wenn die osteokonduktiven Stützkörper tetrapodenförmig gestaltet sind. Eine tetrapodenförmige Gestaltung erlaubt ein besonders wirkungsvolles gegenseitiges Ineinandergreifen der osteokonduktiven Stützkörper.

Weiterhin können die osteokonduktiven Stützkörper, insbesondere Formkörper, längliche Strukturelemente aufweisen. Insbesondere können die osteokonduktiven Stützkörper, insbesondere Formkörper, aus länglichen Strukturelementen zusammengesetzt sein.

Unter dem Ausdruck "längliche Strukturelemente" sollen im Sinne der vorliegenden Erfindung Strukturelemente mit einem Länge-Breite-Verhältnis oder Länge-Durchmesser-Verhältnis > (gesprochen: größer) 1 verstanden werden.

Bevorzugt können die osteokonduktiven Stützkörper, insbesondere Formkörper, längliche und gerade verlaufende Strukturelemente aufweisen. Bevorzugt sind die osteokonduktiven Stützkörper, insbesondere Formkörper, aus länglichen und gerade verlaufenden Strukturelementen zusammengesetzt.

Die länglichen Strukturelemente sind vorzugsweise polyederförmig, insbesondere quaderförmig, würfelförmig, prismenförmig, pyramidenförmig, pyramidenstumpfförmig oder spatförmig, angeordnet. Mit anderen Worten bilden die Strukturelemente eines jeden osteokonduktiven Stützkörpers, insbesondere Formkörpers, vorzugsweise eine polyederförmige, insbesondere quaderförmige, würfelförmige, prismenförmige, pyramidenförmige, pyramidenstumpfförmige oder spatförmige, Anordnung aus.

Die länglichen Strukturelemente können eine Länge von 0,4 mm bis 5 mm, insbesondere 0,8 mm bis 4,5 mm, bevorzugt 1 mm bis 4 mm, aufweisen.

Weiterhin können die länglichen Strukturelemente eine Breite, insbesondere mittlere Breite, oder Durchmesser, insbesondere einen mittleren Durchmesser, von 0,4 mm bis 5 mm, insbesondere 0,8 mm bis 4,5 mm, bevorzugt 1 mm bis 4 mm, aufweisen.

Des Weiteren können die länglichen Strukturelemente einen eckenlosen Querschnitt aufweisen. Beispielsweise können die Strukturelemente einen ovalförmigen, insbesondere kreisförmigen oder elliptischen, Querschnitt aufweisen.

Alternativ können die Strukturelemente einen polygonen Querschnitt aufweisen. Beispielsweise können die Strukturelemente einen dreieckförmigen, quadratförmigen, rechteckförmigen, fünfeckförmigen, sechseckförmigen, siebeneckförmigen, achteckförmigen, neuneckförmigen, zehneckförmigen oder sternenförmigen Querschnitt aufweisen.

Osteokonduktive Stützkörper, insbesondere in Form von Formkörpern, mit länglichen und insbesondere gerade verlaufenden Strukturelementen, insbesondere wie bislang beschrieben, haben den Vorteil, dass durch die gegenseitige Anordnung der Strukturelemente pro Stützkörper, insbesondere Formkörper, zusätzliches Hohlraumvolumen geschaffen werden kann, wodurch sich die osteokonduktiven Eigenschaften der Stützkörper, insbesondere Formkörper, und mithin des Implantats zusätzlich verbessern lassen. Insbesondere können hierdurch die Porengröße (absolutes Hohlraumvolumen) sowie die Porosität (Verhältnis Materialvolumen zu Hohlraumvolumen) von humanem oder tierischem Knochen optimal nachgebildet werden.

In weiterer Ausgestaltung der Erfindung sind die osteokonduktiven Stützkörper unregelmäßig geformt.

Die osteokonduktiven Stützkörper können insbesondere in partikulärer Form, d.h. in Form von Partikeln, vorliegen.

Vorzugsweise sind die osteokonduktiven Stützkörper als gebrochenes Material, insbesondere als Schüttgut, bevorzugt Granulat, gestaltet.

Unter dem Ausdruck "Schüttgut" soll im Sinne der vorliegenden Erfindung ein partikuläres Material, d.h. ein Material in Form von Partikeln, verstanden werden, dessen Partikel wenigstens eine Abmessung oder Dimension kleiner als 7 mm, vorzugsweise in einem Größenbereich von 0,5 mm bis 5 mm, aufweisen. Bei der wenigstens einen Abmessung bzw. Dimension kann es sich um die Höhe und/oder Länge und/oder Breite (Dicke) und/oder den Durchmesser, insbesondere mittleren Durchmesser, der Partikel handeln. Unter dem Ausdruck "Granulat" soll im Sinne der vorliegenden Erfindung ein unregelmäßig geformtes, partikuläres Material, insbesondere ein gebrochenes und/oder gesiebtes Material, verstanden werden. Weiterhin können die osteokonduktiven Stützkörper als nicht gebrochenes Material gestaltet sein. Beispielsweise können die osteokonduktiven Stützkörper als additiv gefertigtes Material, d.h. als Material, welches mittels eines additiven Fertigungsverfahrens hergestellt ist, gestaltet sein.

Unter dem Ausdruck "additives Fertigungsverfahren" soll ein urformendes Verfahren zur schnellen und kostengünstigen Fertigung von Modellen, Mustern, Prototypen, Werkzeugen und Endprodukten verstanden werden, wobei die Fertigung direkt auf der Basis von rechnerinternen Datenmodellen (Übergabe meist über eine STL-Schnittstelle) aus formlosem (beispielsweise Flüssigkeiten, Gelen, Pasten oder Pulver) und formneutralem (beispielsweise bandförmig, drahtförmig oder blattförmig) Material mittels chemischer und/oder physikalischer Prozesse erfolgt. Ein solches Verfahren kann auch als generatives Fertigungsverfahren bezeichnet werden.

Vorzugsweise weisen die osteokonduktiven Stützkörper wenigstens eine Abmessung oder Dimension in einem Größenbereich von 0,5 mm bis 5 mm, insbesondere 0,1 mm bis 3 mm, vorzugsweise 1 mm bis 2 mm, auf. Bei der wenigstens einen Abmessung bzw. Dimension kann es sich um die Höhe und/oder Breite (Dicke) und/oder Länge und/oder den Durchmesser, insbesondere mittleren Durchmesser, der osteokonduktiven Stützkörper handeln.

In weiterer Ausgestaltung der Erfindung sind die osteokonduktiven Stützkörper gegeneinander beweglich, insbesondere gegeneinander verschiebbar, gestaltet.

In weiterer Ausgestaltung der Erfindung sind die osteokonduktiven Stützkörper impaktierbar, d.h. gegenseitig einklemmbar oder gegenseitig verkeilbar, gestaltet.

In weiterer Ausgestaltung der Erfindung liegen die osteokonduktiven Stützkörper impaktiert, d.h. gegenseitig eingeklemmt oder gegenseitig verkeilt, vor.

In weiterer Ausgestaltung der Erfindung sind die osteokonduktiven Stützkörper, bevorzugt mittels Impaktieren, in eine dreidimensionale und insbesondere Hohl- und/oder Zwischenräume aufweisende Struktur oder Matrix überführbar oder liegen in einer solchen Struktur bzw. Matrix vor. Eine solche Struktur bzw. Matrix kann im Sinne der vorliegenden Erfindung auch als osteokonduktive Leitstruktur bzw. osteokonduktive Leitmatrix bezeichnet werden.

Die Hohl- und/oder Zwischenräume der Struktur bzw. Matrix können einen Durchmesser, insbesondere mittleren Durchmesser, von 0,1 mm bis 1,2 mm, insbesondere 0,2 mm bis 1 mm, bevorzugt 0,3 mm bis 0,8 mm, aufweisen.

Weiterhin kann die Struktur bzw. Matrix mit besonderem Vorteil ein Hohl- und/oder Zwischenraumvolumen von 5 % bis 95 %, insbesondere 10 % bis 80 %, bevorzugt 20 % bis 70 %, aufweisen. Ein derartiges Hohl- und/oder Zwischenraumvolumen spiegelt das Porenvolumen einer menschlichen oder tierischen Spongiosa optimal wieder und bewirkt eine Verbesserung der Osteokonduktivität des Implantats sowie insbesondere der biologischen Rekonstruktion eines knöchernen Defekts.

Bevorzugt sind die Hohl- und/oder Zwischenräume der Struktur bzw. Matrix wenigstens zum Teil miteinander verbunden. Auf diese Weise spiegelt die Struktur bzw. die Matrix die Porosität, insbesondere interkonnektierende Porosität, der menschlichen oder tierischen Spongiosa optimal wieder. Dadurch kann mit besonderem Vorteil ebenfalls ein Einwachsen von Knochengewebe in einen defekten Knochenbereich und insbesondere ein Durchwachsen eines defekten Knochenbereichs mit vitalem Knochengewebe angeregt und/oder verstärkt werden. Dies trägt ebenso zu einer Verbesserung der osteokonduktiven Eigenschaften des Implantats und insbesondere der biologischen Rekonstruktion eines Knochendefekts bei.

Weiterhin ist es bevorzugt, wenn die Struktur bzw. Matrix ein Elastizitätsmodul, auch als E-Modul bezeichnet, von 10 MPa bis 10 GPa, insbesondere 50 MPa bis 1 GPa, bevorzugt 80 MPa bis 350 MPa, aufweist. Unter dem Ausdruck "Elastizitätsmodul (E-Modul)" soll im Sinne der vorliegenden Erfindung der Elastizitätsmodul verstanden werden. Der Betrag des Elastizitätsmoduls ist umso größer, je mehr Widerstand ein Material seiner elastischen Verformung entgegensetzt. Ein Körper aus einem Material mit einem hohen Elastizitätsmodul ist daher steifer als ein Körper gleicher Ausgestaltung (gleiche geometrische Abmessung), welcher aus einem Material mit einem niedrigen Elastizitätsmodul besteht. Die in diesem Absatz offenbarten Werte für den Elastizitätsmodul spiegeln in optimaler Weise die entsprechenden Werte von spongiösem Knochen wieder, welcher einen Elastizitätsmodul von 100 MPa bis 1.000 MPa aufweist.

Aufgrund der im vorherigen Absatz beschriebenen geringen E-Module können die osteokonduktiven Stützkörper gleichmäßig, d.h. homogen, mechanisch belastet werden.

Insbesondere können auch die Hohl- und/oder Zwischenhohlräume der in den vorherigen Absätzen beschriebenen Struktur bzw. Matrix mechanisch belastet werden. Durch eine gleichmäßige bzw. homogene mechanische Belastung der osteokonduktiven Stützkörper und mithin des Implantats ist wiederum mit besonderem Vorteil eine Knochenbildung, insbesondere Knochenneubildung, innerhalb eines gesamten knöchernen Defektbereichs erzielbar.

In weiterer Ausgestaltung der Erfindung weisen die osteokonduktiven Stützkörper Öffnungen oder Vertiefungen, insbesondere durchgehende Öffnungen, auf. Dadurch lassen sich die Stützkörper in vorteilhafter Weise bei Belastung (leichter) komprimieren, insbesondere deformieren. Entsprechende zu einer Stützkörperkomprimierung führende Belastungen können beispielsweise bei einer Kraftbeaufschlagung durch einen Anwender, vorzugsweise einen Chirurgen, auftreten. Dadurch lässt sich eine Verdichtung, insbesondere Impaktierung, der osteokonduktiven Stützkörper zusätzlich verbessern, was wiederum verbesserte Tragfähigkeitseigenschaften des Implantats zur Folge hat.

Die Öffnungen bzw. Vertiefungen können ausgewählt sein aus der Gruppe bestehend aus Löcher, Poren, Risse, Schlitze, Ritzen, Spalten, Kerben und Kombinationen aus wenigstens zwei der genannten Öffnungen bzw. Vertiefungen.

Bei den Öffnungen bzw. Vertiefungen kann es sich weiterhin um geometrisch definierte oder undefinierte Öffnungen bzw. Vertiefungen handeln.

Insbesondere können die Öffnungen bzw. Vertiefungen einen ovalen, insbesondere kreisförmigen oder elliptischen, Querschnitt aufweisen. Alternativ oder in Kombination können die Öffnungen bzw. Vertiefungen einen polygonen, insbesondere dreieckförmigen, quadratförmigen, rechteckförmigen, fünfeckförmigen, sechseckförmigen, siebeneckförmigen, achteckförmigen, neuneckförmigen, zehneckförmigen oder sternenförmigen, Querschnitt aufweisen.

Die Öffnungen bzw. Vertiefungen können einen Durchmesser von 0,01 mm bis 5 mm, insbesondere 0,1 mm bis 4 mm, bevorzugt 0,5 mm bis 3 mm, aufweisen. Derartige Durchmesser können bevorzugt sein, wenn die Öffnungen als durchgehende Öffnungen gestaltet sind, durch welche, wie nach folgend noch näher erläutert werden wird, ein Zugelement zum Zwecke des miteinander Verbindens oder Verzurrens der osteokonduktiven Stützkörper hindurch geführt werden soll.

Alternativ können die Öffnungen bzw. Vertiefungen einen Durchmesser, insbesondere mittleren Durchmesser, von 60 µm bis 500 µm, bevorzugt 100 µm bis 400 µm, aufweisen. Derartige Durchmesser sind bevorzugt, wenn die Öffnungen bzw. Vertiefungen als Poren ausgestaltet sind.

Bevorzugt kann es sich bei den Öffnungen bzw. Vertiefungen um Poren handeln. Anders ausgedrückt, können die osteokonduktiven Stützkörper vorzugsweise offenporig gestaltet sein. Insbesondere können die osteokonduktiven Stützkörper eine interkonnektierende Porosität aufweisen.

In weiterer Ausgestaltung der Erfindung weisen die osteokonduktiven Stützkörper Fasern auf. Bei den Fasern kann es sich grundsätzlich um Kurz- und/oder Langfasern handeln.

Unter dem Ausdruck "Kurzfasern" sollen im Sinne der vorliegenden Erfindung Fasern mit einer Länge von 0,01 mm bis 1 mm, insbesondere 0,1 mm bis 1 mm, bevorzugt 0,5 mm bis 1 mm, verstanden werden.

Unter dem Ausdruck "Langfasern" sollen im Sinne der vorliegenden Erfindung Fasern mit einer Länge > (gesprochen: größer) 1 mm verstanden werden.

Bei den Kurz- und/oder Langfasern kann es sich um Metallfasern und/oder Polymerfasern handeln.

In weiterer Ausgestaltung der Erfindung weist das Implantat ferner ein Zugelement auf. Das Zugelement ist vorzugsweise dazu ausgebildet, durch durchgehende Öffnungen der osteokonduktiven Stützkörper hindurch geführt zu werden. Dadurch ist es mit besonderem Vorteil möglich, die osteokonduktiven Stützkörper miteinander zu verbinden oder miteinander zu verzurren. Das Zugelement ist daher zweckmäßigerweise ein längliches Zugelement.

Vorzugsweise handelt es sich bei dem Zugelement um ein textiles, insbesondere fadenförmiges, Zugelement. Beispielsweise kann es sich bei dem Zugelement um einen Faden (Zugfaden), insbesondere um einen monofilen, pseudomonofilen oder multifilen Faden, handeln. Insbesondere kann es sich bei dem Zugelement um einen chirurgischen Nahtfaden handeln.

Weiterhin kann es sich bei dem Zugelement um ein textiles Flächengebilde, insbesondere in Form eines Gewirks, Geflechts, Gestricks, Geleges, Vlies oder Vliesstoffs, handeln. Bevorzugt ist das Zugelement ein Netz, insbesondere ein kleinporiges Netz, vorzugsweise ein Herniennetz. Durch ein Einbinden der osteokonduktiven Stützkörper in ein netzförmiges Zugelement kann eine regelmäßige Anordnung der Stützkörper erzielt werden.

Alternativ kann es sich bei dem Zugelement um einen Draht (Zugdraht) handeln.

Die Verwendung eines Zugelements ermöglicht mit besonderem Vorteil ein Befestigen oder Verzurren der osteokonduktiven Stützkörper, wodurch eine sofortige Festigkeitserhöhung der osteokonduktiven Stützkörper untereinander und damit des Implantats erreicht werden kann. Durch eine derartige Festigkeitserhöhung lässt sich das Risiko senken, dass eine durch die Stützkörper gebildete Gerüststruktur nach einem Sprödbruch auseinanderbricht. Weiterhin kann durch ein Befestigen bzw. Verzurren der osteokonduktiven Stützkörper mit besonderem Vorteil eine offenporige Gerüststruktur realisiert werden. Weiterhin besteht die Möglichkeit, dass eine Zugelement-Stützkörper-Einheit (oder eventuell mehrere Zugelement-Stützkörper-Einheiten) an ein weiteres Implantat und/oder an einen Knochen fixiert und dadurch ortsstabil befestigt werden kann (können). Die Zugelement-Stützkörper-Einheit (oder Zugelement-Stützkörper-Einheiten) kann (können) durch die Befestigung an ein weiteres Implantat beispielsweise an einen angefrischten Knochen angedrückt werden. Hierdurch ist eine optimale Anbindung an den Knochen möglich, und der resultierende Druck auf den Knochen fördert das Knochenwachstum. Die Kraftübertragung am Knochendefekt übernimmt bevorzugt das Implantat. Dadurch entfällt der Druckreiz, welcher den Knochen zum Knochenaufbau anregt (stress shielding). Dieser Druckreiz kann durch das bzw. die unter Druck zum Knochen stehenden Zugelement-Stützkörper-Einheiten aufgebaut werden.

Das Zugelement kann ein Polymer und/oder Metall aufweisen oder aus einem Polymer und/oder Metall bestehen. Bei dem Polymer kann es sich beispielsweise um Polyethylen oder Polypropylen und bei dem Metall beispielsweise um Titan oder Tantal handeln.

In weiterer Ausgestaltung der Erfindung sind die osteokonduktiven Stützkörper derart ausgestaltet, dass sie form-, kraft- und/oder stoffschlüssig miteinander verbindbar sind. Bevorzugt sind die osteokonduktiven Stützkörper derart ausgestaltet, dass sie formschlüssig miteinander verbindbar sind. Beispielsweise können die Stützkörper derart ausgestaltet sein, dass sie über ein Stecksystem oder nach Art eines Stecksystems miteinander verbunden werden können. Das Stecksystem kann dabei auf einem sogenannten Zapfen-Loch-Prinzip beruhen, bevorzugt mit einem Hinterschnitt zur besseren Verankerung der osteokonduktiven Stützkörper. Hierzu können ein Teil der osteokonduktiven Stützkörper mit Zapfen und ein anderer Teil der osteokonduktiven Stützkörper mit passenden Zapflöchern oder Schlitzen versehen sein.

In weiterer Ausgestaltung der Erfindung sind die osteokonduktiven Stützkörper form-, kraft- und/oder stoffschlüssig miteinander verbunden. Bevorzugt sind die Stützkörper formschlüssig miteinander verbunden. Beispielsweise können die osteokonduktiven Stützkörper über ein Stecksystem oder nach Art eines Stecksystems miteinander verbunden sein. Bezüglich weiterer Merkmale und Vorteile des Stecksystems wird auf den vorherigen Absatz Bezug genommen.

In weiterer Ausgestaltung der Erfindung sind die osteokonduktiven Stützkörper derart ausgestaltet, dass sie form-, kraft- und/oder stoffschlüssig mit einem anderen Implantat verbindbar sind. Bevorzugt sind die Stützkörper derart ausgestaltet, dass sie formschlüssig mit einem anderen Implantat verbindbar sind. Beispielsweise können die Stützkörper derart ausgestaltet sein, dass sie über ein Stecksystem oder nach Art eines Stecksystems mit einem Implantat verbunden werden können. Das Stecksystem kann dabei auf einem sogenannten Zapfen-Loch-Prinzip beruhen. Hierzu können die Stützkörper mit einem Zapfen versehen sein, und das andere Implantat kann komplementäre Zapfenlöcher oder Schlitze aufweisen. Die umgekehrten Verhältnisse können erfindungsgemäß ebenso möglich sein.

In weiterer Ausgestaltung der Erfindung sind die osteokonduktiven Stützkörper über längliche Verbindungselemente miteinander verbunden. Bevorzugt ragen die Verbindungselemente hierzu in Ausnehmungen oder Öffnungen der Stützkörper. Bezüglich möglicher Ausgestaltungen der Ausnehmungen oder Öffnungen der Stützkörper wird auf die vorangegangenen Ausführungen Bezug genommen.

In weiterer Ausgestaltung der Erfindung weisen die osteokonduktiven Stützkörper einen Anteil von 10 Gew.-% bis 95 Gew.-%, insbesondere 20 Gew.-% bis 90 Gew.-%, bevorzugt 30 Gew.-% bis 70 Gew.-%, auf, bezogen auf das Gesamtgewicht des Implantats.

In weiterer Ausgestaltung der Erfindung handelt es sich bei dem Implantat um ein Knochenersatzmaterial.

Gemäß einem zweiten Aspekt betrifft die Erfindung ein Kit, vorzugsweise zum Behandeln und/oder biologischen Rekonstruieren, insbesondere Auskleiden und/oder Abdichten und/oder Unterfüttern und/oder wenigstens teilweisen Auffüllen, eines Knochendefekts.

Das Kit weist räumlich getrennt voneinander die folgenden Komponenten auf:
- osteokonduktive Stützkörper und
- eine Umhüllung für die osteokonduktiven Stützkörper.

Das Kit zeichnet sich besonders dadurch aus, dass die Umhüllung ein Umhüllungsmaterial aufweist oder aus einem Umhüllungsmaterial besteht, welches in Wasser oder in einer wasserhaltigen Flüssigkeit löslich ist.

Das chirurgische Kit kann ferner wenigstens eine weitere Komponente aufweisen, welche ausgewählt ist aus der Gruppe bestehend aus Befestigungselemente, künstliche Gelenkpfanne wie künstliche Hüftgelenkpfanne, weiteres biologisches und/oder künstliches Knochenersatzmaterial, Gewebekleber, eine nicht in vivo abbaubare oder nicht in vivo resorbierbare Folie, ein nicht in vivo abbaubares oder nicht in vivo resorbierbares Netz, metallisches Augmentat, ein nicht in vivo abbaubares Vlies oder ein nicht in vivo resorbierbares Vlies, ein nicht in vivo abbaubarer Vliesstoff oder ein nicht in vivo resorbierbarer Vliesstoff, metallische, biegbare oder nicht biegbare Gitterstruktur, Knochenzement, Wachstumsfaktoren und ein oder mehrere Instrumente zum Applizieren einer Gelenkspfanne und/oder eines Stützmaterials.

Bei den Befestigungselementen kann es sich insbesondere um Knochenschrauben oder -nägel handeln.

Bei dem Gewebekleber kann es sich insbesondere um eine aushärtbare Klebstoffzusammensetzung auf Basis von Cyanoacrylat-Monomeren, insbesondere n-Butyl-2-Cyanoacrylat-Monomeren, handeln. Ein solcher Gewebekleber ist zum Beispiel unter der Bezeichnung Histoacryl^{®} kommerziell im Handel erhältlich.

Mittels der bzw. des oben im Zusammenhang einer weiteren Kitkomponente erwähnten in vivo nicht abbaubaren oder in vivo nicht resorbierbaren Folie, Netzes, Vlieses, Vliesstoffes oder einer Kombination, insbesondere einer Kompositstruktur, davon, ist es in vorteilhafter Weise möglich, einen offenen Knochendefekt, insbesondere einen offenen Azetabulumdefekt, auszukleiden, wodurch im Ergebnis die Voraussetzungen für die Behandlung und/oder biologische Rekonstruktion eines geschlossenen Knochendefekts, insbesondere geschlossenen Azetabulumdefekts, geschaffen werden können.

Bezüglich weiterer Merkmale und Vorteile des chirurgischen Kits wird zur Vermeidung von Wiederholungen vollumfänglich auf die im Rahmen des ersten Erfindungsaspekts gemachten Ausführungen Bezug genommen. Die dort insbesondere in Bezug auf die Umhüllung, das Umhüllungsmaterial sowie die osteokonduktiven Stützkörper gemachten Ausführungen gelten (sinngemäß) auch für das chirurgische Kit gemäß zweitem Erfindungsaspekt.

Gemäß einem dritten Aspekt betrifft die Erfindung eine Umhüllung für osteokonduktive Stützkörper. Die Umhüllung weist ein Umhüllungsmaterial auf oder besteht aus einem Umhüllungsmaterial, welches in Wasser oder in einer wasserhaltigen Flüssigkeit löslich ist. Bezüglich weiterer Merkmale und Vorteile der Umhüllung wird zur Vermeidung von unnötigen Wiederholungen vollumfänglich auf die im Rahmen des ersten Erfindungsaspekts gemachten Ausführungen Bezug genommen. Die dort insbesondere in Bezug auf die Umhüllung, das Umhüllungsmaterial sowie die osteokonduktiven Stützkörper gemachten Ausführungen gelten (sinngemäß) auch für die Umhüllung gemäß drittem Erfindungsaspekt.

Gemäß einem vierten Aspekt betrifft die Erfindung die Verwendung eines in Wasser oder in einer wasserhaltigen Flüssigkeit löslichen Materials zur Umhüllung von osteokonduktiven Stützkörpern.

Bezüglich eines geeigneten in Wasser oder einer wasserhaltigen Flüssigkeit löslichen Materials wird auf die im Rahmen des ersten Erfindungsaspekts offenbarten löslichen Umhüllungsmaterialien Bezug genommen. Die dort beschriebenen löslichen Umhüllungsmaterialien kommen auch als Material für die Verwendung gemäß viertem Erfindungsaspekt in Betracht.

Bezüglich weiterer Merkmale und Vorteile der Verwendung wird zur Vermeidung von unnötigen Wiederholungen vollständig auf die im Rahmen des ersten Erfindungsaspekts gemachten Ausführungen Bezug genommen. Die dort insbesondere in Bezug auf die Umhüllung sowie die osteokonduktiven Stützkörper gemachten Ausführungen gelten (sinngemäß) auch für die Verwendung gemäß viertem Erfindungsaspekt.

Ein nicht zur Erfindung gehörendes Beispiel betrifft ein Verfahren zum Behandeln und/oder biologischen Rekonstruieren, insbesondere Auskleiden und/oder Abdichten und/oder Unterfüttern und/oder wenigstens teilweisen Auffüllen, eines Knochendefekts.

Bei dem Knochendefekt handelt es sich vorzugsweise um einen Azetabulumdefekt, insbesondere um einen geschlossenen oder offenen Azetabulumdefekt.

Das Verfahren weist folgenden Schritt auf:
- Platzieren eines Implantats in den Knochendefekt.

Das Verfahren umfasst ferner folgenden Schritt:
- Beaufschlagen des platzierten Implantats mittels eines Nachschlägers, d.h. eines chirurgischen Instruments zum Verdichten, insbesondere Impaktieren, der osteokonduktiven Stützkörper.

Das Verfahren umfasst ferner folgenden Schritt:
- Aufbringen eines Knochenzements auf das platzierte, insbesondere beaufschlagte, Implantat.

In einem weiteren Beispiel weist das Verfahren ferner folgenden Schritt auf:
- Aufbringen einer künstlichen Gelenkpfanne, insbesondere künstlichen Hüftgelenkpfanne, auf die Umhüllung und optional Fixieren/Befestigen der künstlichen Gelenkpfanne, insbesondere künstlichen Hüftgelenkpfanne, an der Umhüllung.

In einem weiteren Beispiel weist das Verfahren ferner folgenden Schritt auf:
- Befestigen der künstlichen Gelenkpfanne, insbesondere künstlichen Hüftgelenkpfanne, an einem Knochen, vorzugsweise an einem den Knochendefekt wenigstens abschnittsweise umgebenden Knochen.

Bezüglich weiterer Merkmale und Vorteile des Verfahrens wird zur Vermeidung von Wiederholungen ebenfalls vollständig auf die im Rahmen des ersten Erfindungsaspekts gemachten Ausführungen Bezug genommen. Die dort in Bezug auf das Implantat, insbesondere die Umhüllung, das Umhüllungsmaterial sowie die osteokonduktiven Stützkörper, gemachten Ausführungen gelten (sinngemäß) auch für das Verfahren das nicht Teil der Erfindung ist.

Weitere Merkmale und Vorteile der Erfindung ergeben sich aus den Ansprüchen sowie aus der nachfolgenden Beschreibung bevorzugter Ausführungsformen anhand eines Ausführungsbeispiels. Dabei können Merkmale der Erfindung jeweils für sich alleine oder in Kombination miteinander verwirklicht sein. Die nachfolgend beschriebenen Ausführungsbeispiele dienen der weiteren Erläuterung der Erfindung, ohne diese hierauf zu beschränken.

### AUSFÜHRUNGSBEISPIEL

### 1. Materialien:

Carboxymethylcellulose (Tylopur C), Polyvinylalkohol (Mowiol 56-98, hochmolar), Polyvinylalkohol (Mowiol 4-98, niedermolar), Wasser für Injektionszwecke (WVI).

### 2. Durchführung:

Zuerst wurden eine 20%ige niedermolare PVA-Lösung und eine 20%ige hochmolare PVA-Lösung in Schottflaschen hergestellt. Hierzu wurden 20 g niedermolekularer Polyvinylalkohol und 80 g Wasser für Injektionszwecke sowie 20 g hochmolekularer Polyvinylalkohol und 80 g Wasser für Injektionszwecke in getrennte Schottflaschen abgefüllt. Anschließend wurden beide Mischungen bis zur vollständigen Lösung in einem Wärmeschrank bei 95 °C für 24 Stunden eingelagert. Danach wurde eine 3%ige Carboxymethylcellulose-Lösung (3 g Natriumcarboxymethylcellulose + 97 g Wasser für Injektionszwecke) in einem Labormischer (Labormischermodell ESCO Typ EL 10) bzw. Homogenisator unter Vakuum (-0,8 bar) bei 35 °C und während einer Mischzeit von etwa 3 bis 4 Stunden hergestellt.

Anschließend wurden eine Mischung aus der niedermolaren PVA-Lösung und der hochmolaren PVA-Lösung, eine Mischung aus der niedermolaren PVA-Lösung und der Carboxymethylcellulose-Lösung sowie eine Mischung aus der hochmolaren PVA-Lösung und der Carboxymethylcellulose hergestellt. Die Herstellung dieser Mischungen erfolgte jeweils mittels des oben genannten Labormischers bzw. Homogenisators (Temperatur maximal 35 °C, Vakuum -0,8 bar, Mischzeit mindestens 2 Stunden).

Die hergestellten Mischungen wurden anschließend mittels eines Labor-Trockners und Fixierers (Mathis Typ LTF 143691) (bei ca. 55 °C bis 60 °C und während einer Trocknungszeit von ca. 25 Minuten) zu Folien gerakelt.

Die Dicke der Folien betrug 38 µm bis 45 µm. Im Wasser lösten sich die Folien innerhalb von 20 s bis 40 s auf.

Je nach Einstellung des Spaltmaßes ließen sich Folien in unterschiedlichen Dicken herstellen. Je dicker die Folien, desto langsamer lösten sich diese in Wasser auf.

Hergestellte Folien mit einer Dicke von 50 µm bis 65 µm lösten sich in Wasser innerhalb von ca. 3 Minuten bis 5 Minuten auf.

Die hergestellten Folien konnten erfolgreich mittels eines Balkenschweißgeräts zu einem Säckchen verschweißt werden, welches zuvor mit osteokonduktiven Stützkörpern gefüllt wurde.

Alternativ zu einem Schweißvorgang ist es denkbar, die Folien mittels eines Gewebeklebers, wie beispielsweise eines Gewebeklebers auf Basis von n-Butyl-2-Cyanoacrylat-Monomeren (Histoacryl^{®}), zu verschließen.

## Patentansprüche

1. Implantat, vorzugsweise zum Behandeln und/oder biologischen Rekonstruieren eines Knochendefekts, aufweisend
- osteokonduktive Stützkörper und
- eine Umhüllung, welche die osteokonduktiven Stützkörper wenigstens teilweise umgibt,
wobei die Umhüllung ein Umhüllungsmaterial aufweist oder aus einem Umhüllungsmaterial besteht, welches in Wasser oder einer wasserhaltigen Flüssigkeit löslich ist **dadurch gekennzeichnet, dass** die Umhüllung Teilbereiche aufweist, welche unterschiedlich gestaltet sind.

2. Implantat nach Anspruch 1, **dadurch gekennzeichnet, dass** das Umhüllungsmaterial ein Polysaccharid aufweist oder aus einem Polysaccharid besteht, welches vorzugsweise ausgewählt ist aus der Gruppe bestehend aus Stärke, Amylose, Amylopektin, Dextran, Methylcellulose, Hydroxymethylcellulose, Ethylcellulose, Hydroxyethylcellulose, Propylcellulose, Hydroxypropylcellulose, Butylcellulose, Hydroxybutylcellulose, Hydroxyethylmethylcellulose, Hydroxypropylmethylcellulose, Carboxymethylcellulose, Hyaluronsäure, Chondroitin-4-sulfat, Chondroitin-6-sulfat, Keratinsulfat, Algininsäure, Heparin, Heparansulfat, Chitosan, Salze davon oder Mischungen davon.

3. Implantat nach Anspruch 1 oder 2, **dadurch gekennzeichnet, dass** das Umhüllungsmaterial ein synthetisches Polymer aufweist oder aus einem synthetischen Polymer besteht, welches vorzugsweise ausgewählt ist aus der Gruppe bestehend aus Polyvinylalkohol, Polyethylenglykol, Ethylenoxid-Propylenoxid-Copolymere (EO-PO-Copolymere), Ethylenoxid-Propylenoxid-Blockcopolymere (EO-PO-Blockcopolymere), Acrylsäure-Homopolymere, Acrylsäure-Copolymere, Polyvinylpyrrolidon-Homopolymere, Polyvinylpyrrolidon-Copolymere oder Mischungen davon.

4. Implantat nach einem der vorhergehenden Ansprüche, **dadurch gekennzeichnet, dass** die Umhüllung als Teilbereiche, welche unterschiedlich gestaltet sind, einen ersten Teilbereich und einen zweiten Teilbereich aufweist.

5. Implantat nach einem der vorhergehenden Ansprüche, **dadurch gekennzeichnet, dass** sich die Teilbereiche in Bezug auf die Löslichkeit der Umhüllung in Wasser oder einer wasserhaltigen Flüssigkeit und/oder in Bezug auf die Dicke der Umhüllung und/oder in Bezug auf die Struktur der Umhüllung voneinander unterscheiden.

6. Implantat nach einem der vorhergehenden Ansprüche, **dadurch gekennzeichnet, dass** sich die Teilbereiche in Bezug auf die chemische Zusammensetzung, insbesondere in Bezug auf eine Vernetzung, des Umhüllungsmaterials voneinander unterscheiden.

7. Implantat nach einem der Ansprüche 4 bis 6, **dadurch gekennzeichnet, dass** der erste Teilbereich ein besser oder schneller in Wasser oder einer wasserhaltigen Flüssigkeit lösliches Umhüllungsmaterial aufweist oder aus einem solchen Umhüllungsmaterial besteht als der zweite Teilbereich.

8. Implantat nach einem der Ansprüche 4 bis 7, **dadurch gekennzeichnet, dass** der erste Teilbereich ein schwächer vernetztes, in Wasser oder einer wasserhaltigen Flüssigkeit lösliches Umhüllungsmaterial aufweist oder aus einem solchen Umhüllungsmaterial besteht als der zweite Teilbereich.

9. Implantat nach einem der Ansprüche 4 bis 8, **dadurch gekennzeichnet, dass** der erste Teilbereich ein nicht vernetztes, in Wasser oder einer wasserhaltigen Flüssigkeit lösliches Umhüllungsmaterial aufweist oder aus einem solchen Umhüllungsmaterial besteht, wohingegen der zweite Teilbereich ein vernetztes, in Wasser oder einer wasserhaltigen Flüssigkeit lösliches Umhüllungsmaterial aufweist oder aus einem solchen Umhüllungsmaterial besteht.

10. Implantat nach einem der Ansprüche 4 bis 9, **dadurch gekennzeichnet, dass** der erste Teilbereich einen höheren Anteil eines in Wasser oder einer wasserhaltigen Flüssigkeit löslichen Umhüllungsmaterial aufweist als der zweite Teilbereich.

11. Implantat nach einem der Ansprüche 4 bis 10, **dadurch gekennzeichnet, dass** der erste Teilbereich ein in Wasser oder einer wasserhaltigen Flüssigkeit lösliches Umhüllungsmaterial aufweist oder aus einem solchen Umhüllungsmaterial besteht, wohingegen der zweite Teilbereich ein in Wasser oder einer wasserhaltigen Flüssigkeit unlösliches Umhüllungsmaterial aufweist oder aus einem solchen Umhüllungsmaterial besteht.

12. Implantat nach einem der Ansprüche 4 bis 11, **dadurch gekennzeichnet, dass** der erste Teilbereich eine kleinere Dicke aufweist als der zweite Teilbereich.

13. Implantat nach einem der Ansprüche 4 bis 12, **dadurch gekennzeichnet, dass** der erste Teilbereich nichttextilförmig, insbesondere folienförmig, und der zweite Teilbereich textilförmig, insbesondere netzförmig, gestaltet ist.

14. Implantat nach einem der Ansprüche 4 bis 13, **dadurch gekennzeichnet, dass** der erste Teilbereich im implantierten Zustand knochenzugewandt und der zweite Teilbereich im implantierten Zustand einer künstlichen Gelenkpfanne, insbesondere künstlichen Hüftgelenkpfanne, zugewandt angeordnet ist.

15. Implantat nach einem der vorhergehenden Ansprüche, **dadurch gekennzeichnet, dass** die osteokonduktiven Stützkörper in vereinzelter Form vorliegen und vorzugsweise Apatit und/oder Tricalciumphosphat aufweisen oder aus Apatit und/oder Tricalciumphosphat und/oder biphasischem Calciumphosphat bestehen.

16. Implantat nach einem der vorhergehenden Ansprüche, **dadurch gekennzeichnet, dass** der Apatit und/oder das Tricalciumphosphat und/oder das biphasische Calciumphosphat eine Porosität von 1 % bis 50 %, insbesondere 5 % bis 20 %, bevorzugt 10 % bis 15 %, aufweist.

17. Implantat nach einem der vorhergehenden Ansprüche, **dadurch gekennzeichnet, dass** der Apatit und/oder das Tricalciumphosphat weitestgehend nicht porös ausgebildet sind.

## Claims

1. Implant, preferably for the treatment and/or biological reconstruction of a bone defect, comprising:
- osteoconductive supporting bodies and
- a sheath that at least partially surrounds the osteoconductive supporting bodies,
the sheath comprising a sheath material or consisting of a sheath material that is soluble in water or in a water-containing liquid, **characterized in that** the sheath has sub-regions that are designed differently.

2. Implant according to claim 1, **characterized in that** the sheath material comprises a polysaccharide or consists of a polysaccharide, which is preferably selected from the group consisting of starch, amylose, amylopectin, dextran, methyl cellulose, hydroxymethyl cellulose, ethyl cellulose, hydroxyethyl cellulose, propyl cellulose, hydroxypropyl cellulose, butyl cellulose, hydroxybutyl cellulose, hydroxyethylmethyl cellulose, hydroxypropylmethyl cellulose, carboxymethyl cellulose, hyaluronic acid, chondroitin-4-sulfate, chondroitin-6-sulfate, keratin sulfate, alginic acid, heparin, heparan sulfate, chitosan, salts thereof, or mixtures thereof.

3. Implant according to claim 1 or 2, **characterized in that** the sheath material comprises a synthetic polymer or consists of a synthetic polymer, which is preferably selected from the group consisting of polyvinyl alcohol, polyethylene glycol, ethylene oxide-propylene oxide copolymers (EO-PO copolymers), ethylene oxide-propylene oxide block copolymers (EO-PO block copolymers), acrylic acid homopolymers, acrylic acid copolymers, polyvinylpyrrolidone homopolymers, polyvinylpyrrolidone copolymers, or mixtures thereof.

4. Implant according to any one of the preceding claims, **characterized in that** the sheath has a first sub-region and a second sub-region as sub-regions that are designed differently.

5. Implant according to any one of the preceding claims, **characterized in that** the sub-regions differ from one another with regard to the solubility of the sheath in water or in a water-containing liquid and/or with regard to the thickness of the sheath and/or with regard to the structure of the sheath.

6. Implant according to any one of the preceding claims, **characterized in that** the sub-regions differ from one another with regard to the chemical composition of the sheath material, in particular with regard to a crosslinking thereof.

7. Implant according to any one of claims 4 to 6, **characterized in that** the first sub-region comprises a sheath material that is more readily soluble or more rapidly soluble in water or in a water-containing liquid than the second sub-region, or consists of such a sheath material.

8. Implant according to any one of claims 4 to 7, **characterized in that** the first sub-region comprises a weaker crosslinked sheath material that is soluble in water or in a water-containing liquid than the second sub-region, or consists of such a sheath material.

9. Implant according to any one of claims 4 to 8, **characterized in that** the first sub-region comprises a non-crosslinked sheath material that is soluble in water or in a water-containing liquid, or consists of such a sheath material, whereas the second sub-region comprises a crosslinked sheath material that is soluble in water or in a water-containing liquid, or consists of such a sheath material.

10. Implant according to any one of claims 4 to 9, **characterized in that** the first sub-region comprises a higher proportion of a sheath material that is soluble in water or in a water-containing liquid than the second sub-region.

11. Implant according to any one of claims 4 to 10, **characterized in that** the first sub-region comprises a sheath material that is soluble in water or in a water-containing liquid, or consists of such a sheath material, whereas the second sub-region comprises a sheath material that is insoluble in water or in a water-containing liquid, or consists of such a sheath material.

12. Implant according to any one of claims 4 to 11, **characterized in that** the first sub-region has a smaller thickness than the second sub-region.

13. Implant according to any one of claims 4 to 12, **characterized in that** the first sub-region is of non-textile form, in particular film form, and the second sub-region is of textile form, in particular mesh form.

14. Implant according to any one of claims 4 to 13, **characterized in that** in the implanted state the first sub-region is arranged facing toward a bone, and in the implanted state the second sub-region is arranged facing toward an artificial socket, in particular an artificial hip joint socket.

15. Implant according to any one of the preceding claims, **characterized in that** the osteoconductive supporting bodies are in individual form and preferably comprise apatite and/or tricalcium phosphate or consist of apatite and/or tricalcium phosphate and/or biphasic calcium phosphate.

16. Implant according to any one of the preceding claims, **characterized in that** the apatite and/or the tricalcium phosphate and/or the biphasic calcium phosphate has a porosity of 1% to 50%, in particular 5% to 20%, preferably 10% to 15%.

17. Implant according to any one of the preceding claims, **characterized in that** the apatite and/or the tricalcium phosphate are largely non-porous.

## Revendications

1. Implant, de préférence pour le traitement et/ou la reconstruction biologique d'un défaut osseux, présentant
- des corps supports ostéoconducteurs et
- une enveloppe qui entoure au moins partiellement le corps support ostéoconducteur,
l'enveloppe présentant un matériau d'enveloppe ou se composant d'un matériau d'enveloppe qui est soluble dans l'eau ou dans un liquide contenant de l'eau, **caractérisé en ce que** l'enveloppe présente des zones partielles qui sont conçues différemment.

2. Implant selon la revendication 1, **caractérisé en ce que** le matériau d'enveloppe présente un polysaccharide ou se compose d'un polysaccharide qui est choisi de préférence dans le groupe constitué d'amidon, d'amylose, d'amylopectine, de dextrane, de méthylcellulose, d'hydroxyméthylcellulose, d'éthylcellulose, d'hydroxyéthylcellulose, de propylcellulose, d'hydroxypropylcellulose, de butylcellulose, d'hydroxybutylcellulose, d'hydroxyéthylméthylcellulose, d'hydroxypropyl-méthylcellulose, de carboxyméthylcellulose, d'acide hyaluronique, de 4-sulfate de chondroïtine, de 6-sulfate de chondroïtine, de sulfate de kératine, d'acide alginique, d'héparine, de sulfate d'héparane, de chitosane, de sels de ceux-ci ou mélanges de ceux-ci.

3. Implant selon la revendication 1 ou 2, **caractérisé en ce que** le matériau d'enveloppe présente un polymère synthétique ou se compose d'un polymère synthétique, qui est choisi de préférence dans le groupe constitué d'alcool polyvinylique, de polyéthylène glycol, de copolymères d'oxyde d'éthylène - d'oxyde de propylène (copolymères OE-OP), de copolymères séquencés d'oxyde d'éthylène - d'oxyde de propylène (copolymères séquencés OE-OP), d'homopolymères d'acide acrylique, de copolymères d'acide acrylique, d'homopolymères de polyvinylpyrrolidone, de copolymères de polyvinylpyrrolidone ou de mélanges de ceux-ci.

4. Implant selon l'une des revendications précédentes, **caractérisé en ce que** l'enveloppe présente une première zone partielle et une deuxième zone partielle en tant que zones partielles qui sont conçues différemment.

5. Implant selon l'une des revendications précédentes, **caractérisé en ce que** les zones partielles se distinguent les unes des autres au niveau de la solubilité de l'enveloppe dans l'eau ou dans un liquide contenant de l'eau et/ou au niveau de l'épaisseur de l'enveloppe et/ou au niveau de la structure de l'enveloppe.

6. Implant selon l'une des revendications précédentes, **caractérisé en ce que** les zones partielles se distinguent les unes des autres au niveau de la composition chimique, notamment au niveau d'une réticulation, du matériau d'enveloppe.

7. Implant selon l'une des revendications 4 à 6, **caractérisé en ce que** la première zone partielle présente un matériau d'enveloppe mieux soluble ou plus rapidement soluble dans l'eau ou dans un liquide contenant de l'eau ou se compose d'un tel matériau d'enveloppe en tant que deuxième zone partielle.

8. Implant selon l'une des revendications 4 à 7, **caractérisé en ce que** la première zone partielle présente un matériau d'enveloppe réticulé plus faiblement, soluble dans l'eau ou dans un liquide contenant de l'eau ou se compose d'un tel matériau d'enveloppe en tant que deuxième zone partielle.

9. Implant selon l'une des revendications 4 à 8, **caractérisé en ce que** la première zone partielle présente un matériau d'enveloppe non réticulé, soluble dans l'eau ou dans un liquide contenant de l'eau ou se compose d'un tel matériau d'enveloppe en tant que deuxième zone partielle, alors que la deuxième zone partielle présente un matériau d'enveloppe réticulé, soluble dans l'eau ou dans un liquide contenant de l'eau ou se compose d'un tel matériau d'enveloppe.

10. Implant selon l'une des revendications 4 à 9, **caractérisé en ce que** la première zone partielle présente une proportion plus élevée d'un matériau d'enveloppe soluble dans l'eau ou dans un liquide contenant de l'eau en tant que deuxième zone partielle.

11. Implant selon l'une des revendications 4 à 10, **caractérisé en ce que** la première zone partielle présente un matériau d'enveloppe soluble dans l'eau ou dans un liquide contenant de l'eau ou se compose d'un tel matériau d'enveloppe alors que la deuxième zone partielle présente un matériau d'enveloppe insoluble dans l'eau ou dans un liquide contenant de l'eau ou se compose d'un tel matériau d'enveloppe.

12. Implant selon l'une des revendications 4 à 11, **caractérisé en ce que** la première zone partielle présente une épaisseur plus petite que la deuxième zone partielle.

13. Implant selon l'une des revendications 4 à 12, **caractérisé en ce que** la première zone partielle n'est pas conçue sous la forme d'un textile, est conçue notamment sous la forme d'un film, et que la deuxième zone partielle est conçue sous la forme d'un textile, notamment d'un filet.

14. Implant selon l'une des revendications 4 à 13, **caractérisé en ce que** la première zone partielle est disposée tournée vers l'os à l'état implanté et la deuxième zone partielle est disposée tournée vers une cupule acétabulaire artificielle à l'état implanté, notamment une cupule acétabulaire artificielle de hanche.

15. Implant selon l'une des revendications précédentes, **caractérisé en ce que** les corps supports ostéoconducteurs se présentent sous une forme individualisée et de préférence présentent de l'apatite et/ou du phosphate tricalcique ou se composent d'apatite et/ou de phosphate tricalcique et/ou de phosphate de calcium biphasique.

16. Implant selon l'une des revendications précédentes, **caractérisé en ce que** l'apatite et/ou le phosphate tricalcique et/ou le phosphate de calcium biphasique présente une porosité de 1 % à 50 %, en particulier de 5 % à 20 %, de préférence de 10 % à 15 %.

17. Implant selon l'une des revendications précédentes, **caractérisé en ce que** l'apatite et/ou le phosphate tricalcique sont conçus le plus largement possible non poreux.
